# EUROPEAN PATENT SPECIFICATION

(11) **EP 4 392 100 B1**
(45) Date of publication and mention of the grant of the patent: **23.07.2025**
(21) Application number: 22790120.4
(22) Date of filing: 21.09.2022
(51) Int. Cl.: A61M 5/28, A61J 1/06

(54) **PRE-FILLED BLOW-FILL-SEAL INTRADERMAL INJECTION SYSTEM**
INTRADERMALES INJEKTIONSSYSTEM MIT VORGEFÜLLTEM BLASFÜLLVERSCHLUSS
SYSTÈME D'INJECTION INTRADERMIQUE À FORMAGE-REMPLISSAGE-SCELLAGE PRÉ-REMPLI

(30) Priority: 21.09.2021 US 202163246759 P
(43) Date of publication of application: 03.07.2024
(73) Proprietor: Koska Family Limited, East Sussex BN21 4PT (GB)
(72) Inventor: KOSKA, Marc, East Sussex Eastbourne Sussex BN21 4PT (GB); HANNON, Max, Flemingate Beverley HU17 0TU (GB); PRICE, Jeff, Windermere, FL 34786 (US)
(74) Representative: Mewburn Ellis LLP
(86) International application number: PCT/US2022/044291
(87) International publication number: WO 2023/049213

(56) References cited:
- WO-A1-2015/187518
- WO-A1-2019/246435
- WO-A2-2010/081174
- US-A1- 2014 323 975
- US-A1- 2015 165 123

## Description

### CROSS-REFERENCE TO RELATED APPLICATIONS

This application claims benefit and priority to, and is a Non-provisional of, U.S. Provisional Patent Application No. 63/246,759 filed on September 21, 2021 and titled "SYSTEMS AND METHODS FOR BLOW-FILL-SEAL (BFS) INTRADERMAL INJECTION".

### BACKGROUND

While IntraDermal ("ID") injections are preferable for the administration of various medicaments, difficulties in administration and in providing effective injection devices have limited the practical application of ID injections. US 2015/165123 A1 proposes a single use injector. WO 2019/246435 A1 proposes a system and method for dual-component drug agent delivery. WO 2010/081174 A2 proposes packaged products, inserts, and compartments for aseptic mixing of substances. WO 2015/187518 A1 proposes a prefilled disposable injection device. US 2014/323975 A1 proposes a prefilled medical injection device.

### Summary of Invention

In a first aspect, embodiments of the invention provide a pre-filled blow-fill-seal intradermal injection system as set out in claim 1.

### BRIEF DESCRIPTION OF THE DRAWINGS

An understanding of embodiments described herein and many of the attendant advantages thereof may be readily obtained by reference to the following detailed description when considered with the accompanying drawings, wherein:
FIG. 1A, FIG. 1B, FIG. 1C, FIG. 1D, FIG. 1E, FIG. 1F, FIG. 1G, FIG. 1H, FIG. 11, FIG. 1J, FIG. 1K, FIG. 1L, and FIG. 1M are various views of a pre-filled BFS ID injection system not according to the claimed invention;
FIG. 2A, FIG. 2B, FIG. 2C, FIG. 2D, FIG. 2E, FIG. 2F, FIG. 2G, FIG. 2H, and FIG. 2I are front perspective, rear perspective, front perspective cross-section, front, back, left, right, top, and bottom views of an ID injection hub not according to the claimed invention;
FIG. 3A, FIG. 3B, FIG. 3C, FIG. 3D, FIG. 3E, FIG. 3F, FIG. 3G, and FIG. 3H are front perspective, perspective cross-section, front, back, left, right, top, and bottom views of a BFS ID injection connector not according to the claimed invention;
FIG. 4A, FIG. 4B, FIG. 4C, FIG. 4D, FIG. 4E, FIG. 4F, FIG. 4G, and FIG. 4H are front perspective, perspective cross-section, front, back, left, right, top, and bottom views of an ID injection shield not according to the claimed invention;
FIG. 5A, FIG. 5B, FIG. 5C, and FIG. 5D are front perspective, front, top, and bottom views of pre-filled BFS vial not according to the claimed invention;
FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N are various views of a pre-filled BFS ID injection system according to some embodiments;
FIG. 7A, FIG. 7B, FIG. 7C, FIG. 7D, FIG. 7E, FIG. 7F, FIG. 7G, and FIG. 7H are front left perspective, top left perspective, top right perspective, bottom left perspective, front, left, top, and bottom views of an ID injection shield according to some embodiments;
FIG. 8A, FIG. 8B, FIG. 8C, FIG. 8D, FIG. 8E, and FIG. 8F are top left perspective, bottom left perspective, front, left, top, and bottom views of a BFS ID injection piston according to some embodiments;
FIG. 9A, FIG. 9B, FIG. 9C, FIG. 9D, FIG. 9E, and FIG. 9F are top left perspective, bottom left perspective, front, left, top, and bottom views of an ID injection hub according to some embodiments;
FIG. 10A, FIG. 10B, FIG. 10C, FIG. 10D, FIG. 10E, and FIG. 10F are bottom left perspective, side, top, bottom, side cross-section, and perspective side cross-section views of a BFS ID injection connector according to some embodiments; and
FIG. 11A and FIG. 11B are top and bottom perspective views of a BFS ID injection cap according to some embodiments.

### DETAILED DESCRIPTION

### I. Introduction

Embodiments of the present invention provide systems and methods (not claimed) for pre-filled, single-dose, and/or IntraDermal ("ID") medical agent delivery that overcome drawbacks of current delivery devices and methods. For example, the pre-filled, single-dose, and/or ID medical delivery systems or assemblies of some embodiments may include a plastic (*e.g.,* a Blow-Fill-Seal (BFS)) vial or bottle coupled to a specialized collar, coupling, or connector that facilitates coupling of an administration member (*e.g.,* an ID needle/canula) to the BFS vial. In some embodiments, such a pre-filled, single-dose, and/or ID medical delivery assembly may be selectively actuated by application of an axial or longitudinal force to a shield and/or piston covering the administration member, causing the administration member to axially advance and pierce a fluid reservoir of the BFS vial. In some embodiments, a specialized BFS coupling, connector, or collar may be utilized to seat the BFS vial in a position for puncturing. According to some embodiments, a pre-filled BFS ID delivery system may comprise one or more auto-disable features that permit the system to be utilized for a single dose delivery, after which the system becomes inoperable to administer additional doses (*e.g.,* preventing needle reuse). In some embodiments, the disabling of the device may be accomplished by providing an eccentrically-disposed needle/canula that becomes automatically misaligned with an administration port following use. According to some embodiments, the device may be self-actuating in response to applied axial/longitudinal force such as by employing a plurality of elastic legs that engage with cooperative beveled and/or angled surfaces to displace the legs, thereby imparting an elastic deformation force to the internal components of the device.

### II. Pre-filled BFS ID Medical Agent Delivery Systems - Six (6) Component

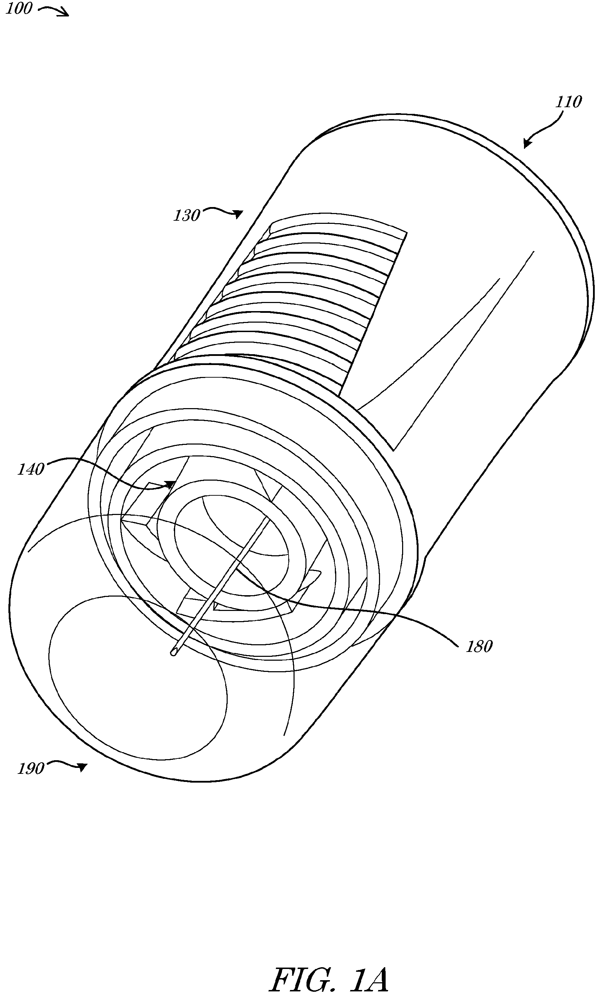

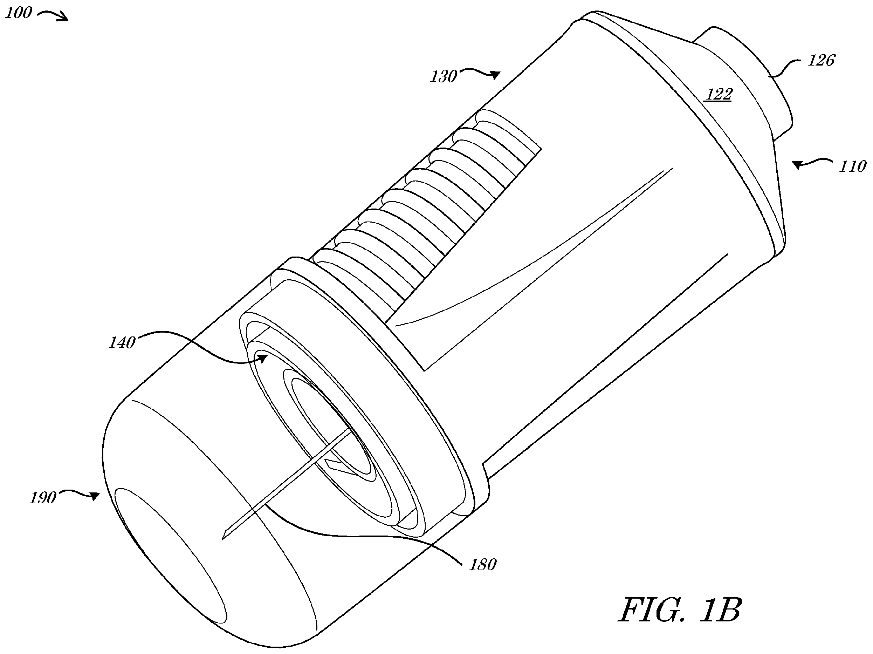

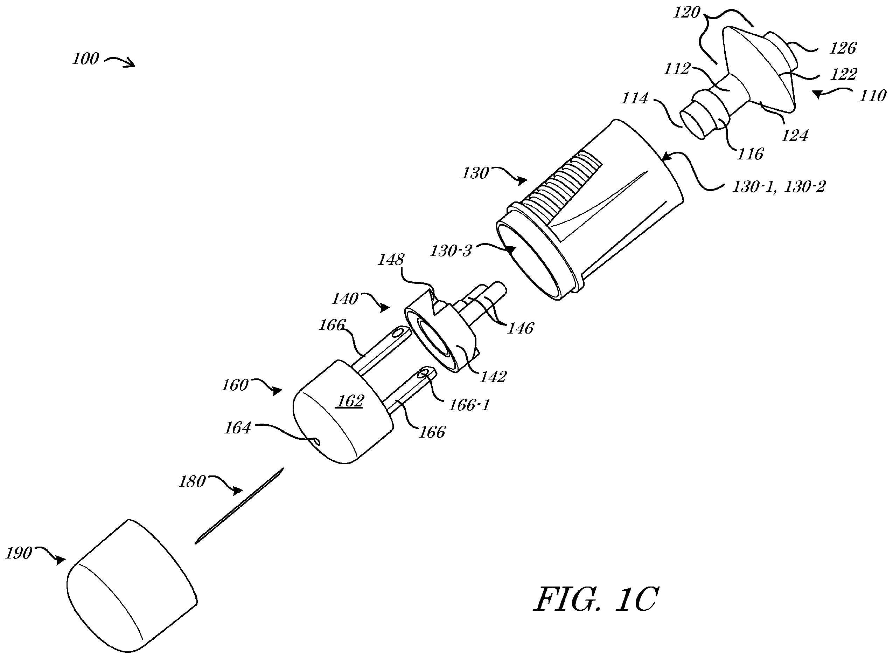

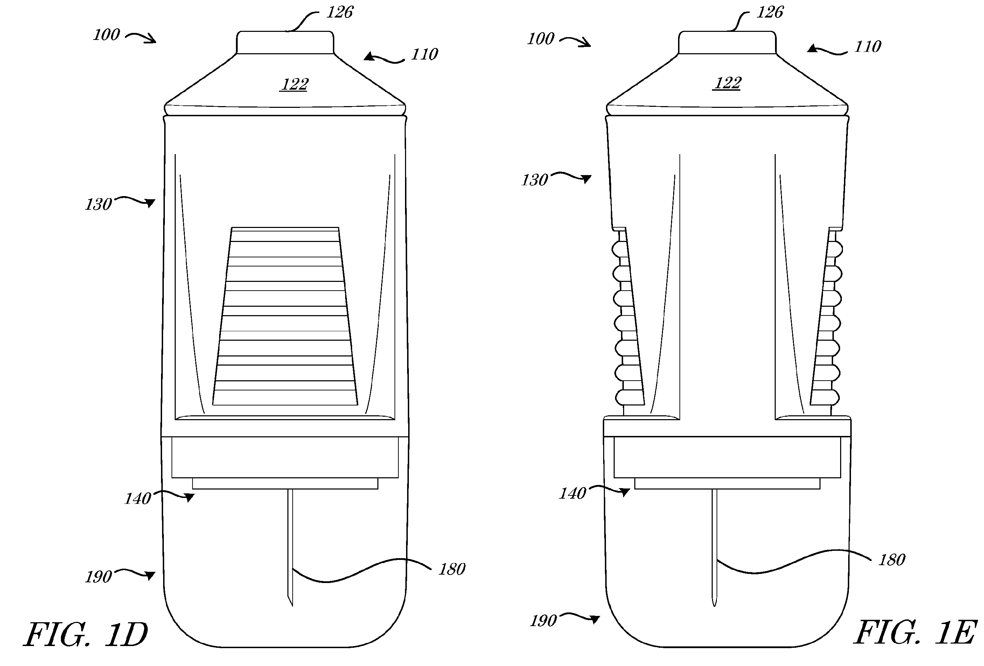

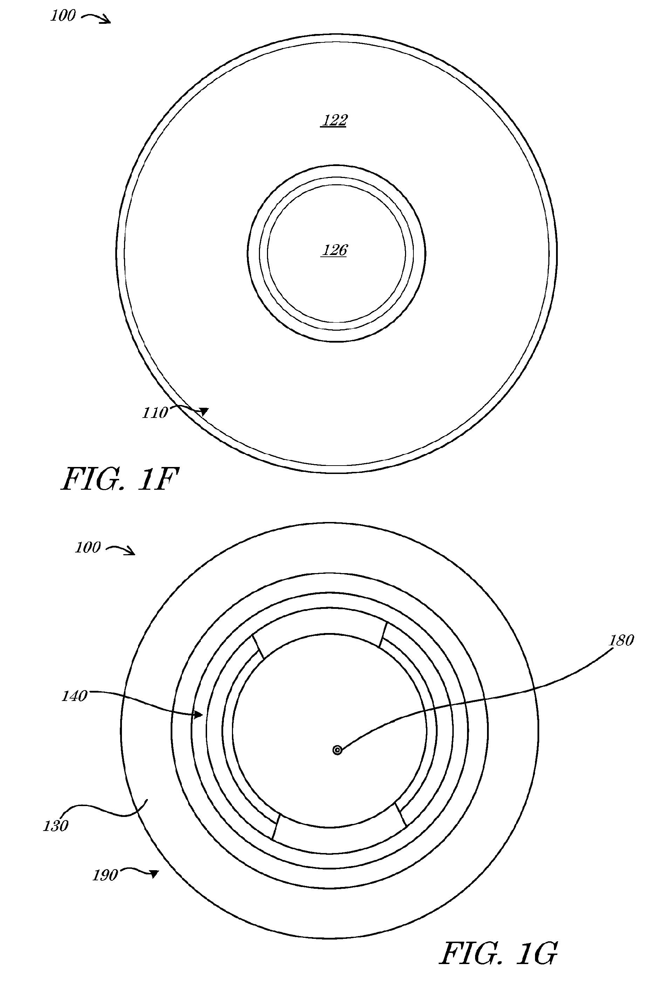

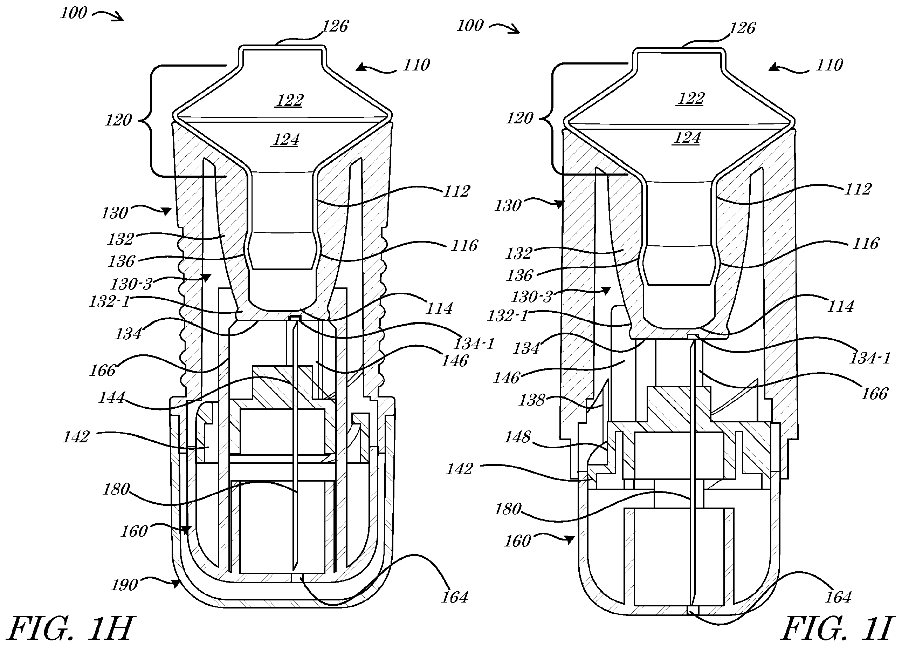

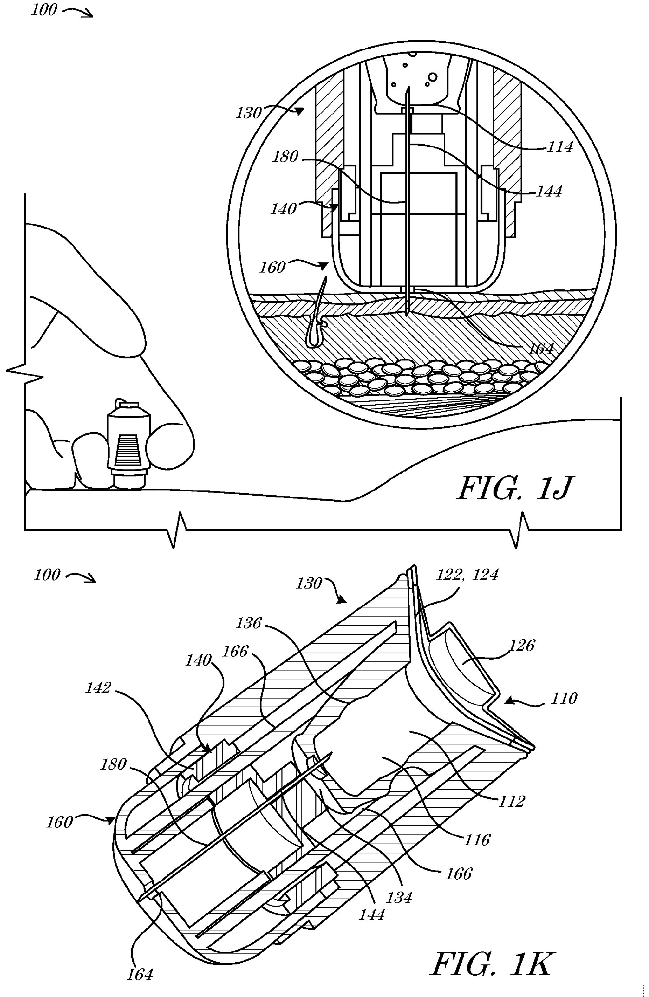

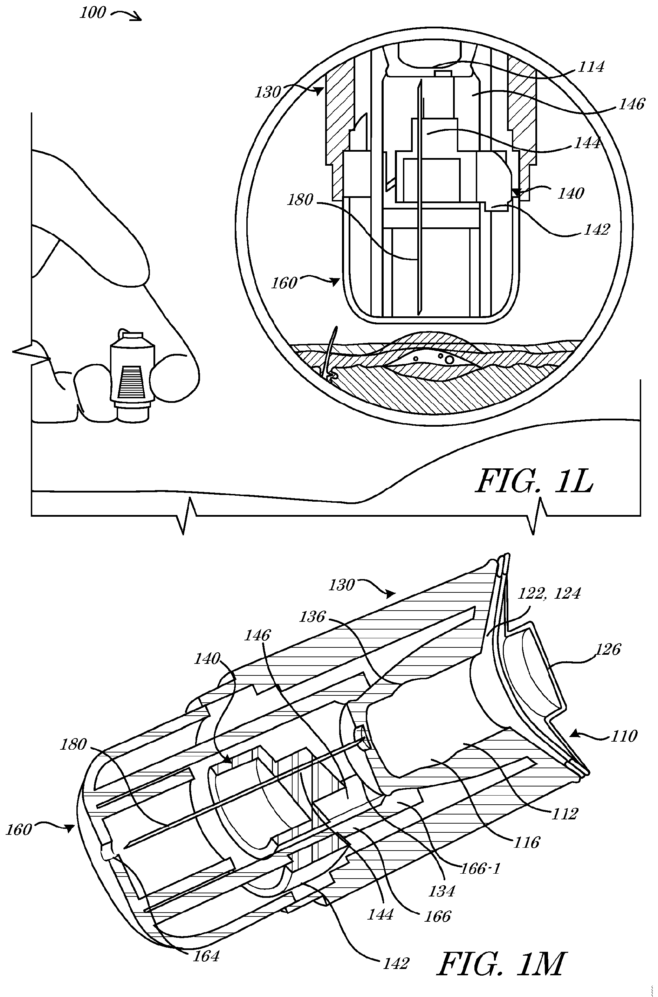

Referring initially to FIG. 1A, FIG. 1B, FIG. 1C, FIG. 1D, FIG. 1E, FIG. 1F, FIG. 1G, FIG. 1H, FIG. 1I, FIG. 1J, FIG. 1K, FIG. 1L, and FIG. 1M, various views of a pre-filled BFS ID injection system 100. The pre-filled BFS ID injection system 100 may comprise various inter-connected and/or modular components such as a BFS vial 110 (or bottle, unit, ampule, etc.) comprising and/or defining a vial neck 112, a fluid seal 114, a mounting flange 116, and/or a collapsible reservoir 120 (*e.g.,* comprising an upper reservoir surface 122, a lower reservoir surface 124, and/or a push surface 126). The BFS vial 110 may comprise a plastic and/or synthetic vial that is constructed via any practicable manufacturing techniques. The BFS manufacturing technique may be utilized. Where the BFS vial 110 is constructed utilizing BFS manufacturing techniques, special characteristics may necessarily be imparted to the BFS vial 110 due to the BFS manufacturing technique and may accordingly be inherent in the BFS vial 110. Such characteristics may include, but are not limited to (and indeed may not require), the BFS vial 110 (i) being manufactured in an aseptic and/or sterile environment, (ii) being automatically filled and sealed in the aseptic/sterile environment, (iii) being comprised of a soft plastic, *e.g.,* having a Shore/Durometer "D" hardness of between 60 and 70, (iv) being formed in two conjoined halves along an axial seam, (v) being geometrically limited to symmetrically moldable features, and/or (vi) being limited to formation in particular temperature ranges (*e.g.,* above freezing). While a particular shape and configuration of the BFS vial 110 is depicted for ease of illustration and explanation, other shapes and/or configurations may be utilized. The BFS vial 110 may not include, for example, the mounting flange 116 and/or may comprise a side or seam flange (not shown; *e.g.,* formed between different co-manufactured units of BFS vials 110 and providing and/or defining a cut or punch surface at which the different units are separated post-manufacture).

The pre-filled BFS ID injection system 100 may comprise a mounting collar or connector 130 in which the BFS vial 110 is seated. The connector 130 may comprise and/or define, for example, a BFS chamber 130-1, a BFS detent 130-2, a hub bore 130-3, an interior cone 132, a cone lip 132-1, an interior surface 134, a seat 136, and/or a track 138. The pre-filled BFS ID injection system 100 may comprise a hub 140 comprising an annular, circular, and/or cylindrical base 142, an administration seat 144, one or more legs 146, one or more feet 146-1, and/or one or more rails 148. The pre-filled BFS ID injection system 100 may comprise an injection shield 160 comprising a cylindrical body 162, an administration bore 164, and/or one or more legs 166 and/or one or more feet 166-1. The pre-filled BFS ID injection system 100 may comprise an administration member 180 and/or a cap 190. The pre-filled BFS ID injection system 100 may comprise a modular design consisting of separately constructed components 110, 130, 140, 160, 180, 190 cooperatively arranged and coupled to one another. Some of the components 110, 130, 140, 160, 180, 190 may be manufactured, created, molded, and/or otherwise formed together.

The collapsible reservoir 120 of the BFS vial 110 may be filled (fully or partially) with a fluid or other agent (not separately shown) such as a medicament. The fluid may be injected into the BFS vial 110 in a sterile environment during manufacture via a BFS process and sealed within the BFS vial 110 via the fluid seal 114. The fluid seal 114 may comprise a portion of the molded BFS vial 110 for example that is configured to be pierced to expel the fluid, *e.g.,* such as by providing a flat or planar piercing surface, being selectively formed with a particular material thickness, and/or by being oriented normal to an axis of the BFS vial 110 (and/or the pre-filled BFS ID injection system 100). The fluid seal 114 may comprise a foil, wax, paper, and/or other thin, pierceable object or layer coupled to the BFS vial 110. The neck 112 of the BFS vial 110 may comprise the mounting flange 116 such as, e.g., the "doughnut"-shaped exterior flange depicted (and/or one or more other tabs, detents, protrusions, and/or other features). The mounting flange 116 may also or alternatively comprise a side or seam flange (not depicted), e.g., whether disposed/formed on the neck 112 or elsewhere on the BFS vial 110. As depicted, the neck 112 may be cylindrically shaped. The neck 112 may comprise one or more other cross-sectional shapes or configurations such as a triangle, square, rectangle, pentagon, hexagon, star, and/or octagon shape. The shape of the neck 112 may correspond to a type of fluid agent stored in the BFS vial 110.

The connector 130 may be axially engaged to couple with the BFS vial 110 via application of an axial mating force. The connector 130 (and/or the BFS chamber 130-1 thereof) may be urged onto the neck 112 of the BFS vial 110, for example (or *vice versa*), such that it accepts and/or selectively couples to the mounting flange 116 (e.g., via the seat 136), thereby removably coupling the BFS vial 110 and the mounting collar 130. The BFS chamber 130-1 (and/or the BFS detent 130-2) may be shaped to correspond to and/or cooperatively mate with the shape of the neck 112 and/or of the collapsible reservoir 120 of the BFS vial 110. In the case that the neck 112 is cylindrically or triangularly shaped, for example, the BFS chamber 130-1 may comprise a cylindrical or triangular opening and/or passage, respectively. Uncoupling of the BFS vial 110 and the connector 130 may be mechanically prohibited. The mounting flange 116 may effectively lock into the seat 136 connector 130 once inserted, for example, preventing or inhibiting removal thereafter. When seated in the BFS chamber 130-1 (and/or the BFS detent 130-2), the BFS vial 110 may be substantially shrouded by the connector 130. As depicted, for example, in a seated configuration, only the upper reservoir surface 122 and the push surface 126 may be externally exposed and/or accessible. A first end of the connector 130 (and/or of the pre-filled BFS ID injection system 100) may be referred to as a distal end, as it is distal from a site of injection (not separately labeled). Utilizing such terminology, the BFS vial 110 may be coupled to and/or seated in the connector 130 at the distal end thereof.

The hub 140 may be selectively coupled to and/or seated in the hub bore 130-3 of a proximal end of the connector 130. The legs 146 of the hub 140 may extend into the hub bore 130-3 and engage with the interior cone 132 and/or the cone lip 132-1 thereof. Each leg 146 (and/or a foot 146-1 thereof) may comprise a pliable member that is elastically and radially offset as the hub 140 is inserted axially deeper into the hub bore 130-3 such that the legs 146 (and/or feet 146-1 thereof) travel along the increasing outer diameter of the interior cone 132. In such a manner, for example, elastic and/or axial resistance may increase as the hub 140 is inserted axially deeper into the hub bore 130-3. The hub 140 may couple to and/or retain the administration member 180. The administration member 180 may be inserted into the administration seat 144 of the hub 140, for example, such that a first or piercing end (not separately labeled) is disposed within the hub bore 130-3, e.g., in the case that the hub 140 is seated in the hub bore 130-3, and a second or administration end (not separately labeled) extends axially distal from the BFS vial 110. In a first state or engagement position, the administration member 180 and/or the piercing end thereof may be positioned adjacent to (e.g., axially adjacent to) the interior surface 134 (and/or a specific portion thereof). The administration end and/or a distal portion of the administration member 180 may be housed, shrouded, and/or covered by the shield 160 and/or the cap 190.

When seated in the hub bore 130-3, the rails 148 of the hub 140 may engage with and/or seat in the track 138. As depicted, the track 138 and corresponding rails 148 may be shaped in a triangular and/or otherwise cammed or disjointed track pattern such that axial insertion of the hub 140 into the hub bore 130-3 causes an axial rotation of the hub 140 with respect to the connector 130. Such a rotation may cause the administration member 180 and/or the piercing end thereof to attain a piercing position with respect to the interior surface 134 (and/or a specific portion thereof). As depicted, for example, the administration member 180 (and the corresponding administration seat 144) may be axially offset (e.g., eccentrically positioned) from a central axis of the pre-filled BFS ID injection system 100. Rotation of the hub 140 may cause the piercing end of the administration member 180 to travel along a circular path over or on the interior surface 134. In the case that a particular portion of the interior surface 134 is thinner and/or otherwise configured to accept the administration member 180, the administration member 180 may pierce the interior surface 134 and travel into and pierce the fluid seal 114 of the BFS vial 110 upon achievement of a particular axial insertion distance (and corresponding axial rotation due to engagement of the track 138 and rails 148). The pre-filled BFS ID injection system 100 may be configured such that a rotation in the range of three degrees (3°) to seven degrees (7°) of the hub 140 (and/or the shield 160) may cause the administration member 180 to become misaligned with the administration bore 164 and/or a desired portion of the interior surface 134 (e.g., a puncture area - not separately shown or labeled).

The shield 160 may be selectively coupled to and/or seated in the hub bore 130-3 of the proximal end of the connector 130. The legs 166 of the shield 160 may extend into the hub bore 130-3 and engage with the interior cone 132 and/or the cone lip 132-1 thereof. Each leg 166 (and/or a foot 166-1 thereof) may comprise a pliable member that is elastically and radially offset as the shield 160 is inserted axially deeper into the hub bore 130-3 such that the legs 166 (and/or feet 166-1 thereof) travel along the increasing outer diameter of the interior cone 132. In such a manner, for example, elastic and/or axial resistance may increase as the shield 160 is inserted axially deeper into the hub bore 130-3. As depicted, each of the hub 140 and the shield 160 may be coupled to the connector 130. The legs 166 of the shield 160 may, for example, extend through and/or past the hub 140 such that all legs 146, 166 (and/or feet 146-1, 166-1) simultaneously engage with the interior cone 132. The number, size, and/or shape of the legs 146, 166 (and/or feet 146-1, 166-1) and the angle of the interior cone 132 may be adjusted to achieve a desired axial insertion resistance profile.

As depicted in FIG. 1J, the shield 160 and the hub 140 may be axially advanced into the hub bore 130-3 by engaging (*e.g.,* pressing) the proximal end of the shield 160 against an injection surface (*e.g.,* human skin). Such advancement/engagement may cause the piercing end of the administration member 180 to advance through the interior surface 134 (and/or a specific portion thereof and pierce the fluid seal 114 of the BFS vial 110. The advancement/engagement may also cause the administration end of the administration member 180 to become aligned with and advance through the administration bore 164 and into the target. Once the administration member 180 has entered the target, the push surface 126 of the BFS vial 110 may be axially forced toward the injection site, causing the collapsible reservoir 120 to compress against the BFS detent 130-2 and thereby causing any fluid therein to advance through the administration member 180 and into the target (*e.g.,* forming an ID "bleb"). Release of the axial pressure/force from the shield 160 may cause the elastic force of the legs 146, 166 (and/or feet 146-1, 166-1) to release, thereby causing the legs 146, 166 (and/or feet 146-1, 166-1) to retreat from the interior cone 132. The legs 146, 166 (and/or feet 146-1, 166-1) may stop retreating upon engagement with the cone lip 132-1, e.g., preventing uncoupling of the hub 140 and the shield 160 from the connector 130.

The reverse travel or retreating of the legs 146, 166 (and/or feet 146-1, 166-1) may cause the rails 148 riding in the track 138 to urge the hub 140 (and/or the shield 160) to rotate axially. Such rotation may cause the axially-offset administration member 180 to become misaligned from the hole made by the piercing of the BFS vial 110 and/or from the administration bore 164. In such a manner, for example, the pre-filled BFS ID injection system 100 may become deactivated and/or rendered unusable (e.g., for reuse). This rotational misalignment and/or the collapsing of the collapsible reservoir 120 may selectively and automatically disable the pre-filled BFS ID injection system 100.

Hub 140 and shield 160 combination may be utilized to couple and/or mate the administration member 180 with the connector 130. The connector 130 may provide a mechanism via which the administration member 180 may be coupled to be in fluid communication with a soft plastic BFS vial 110 in a reliable manner. Due to the nature of the BFS plastic and/or process and/or the small form-factor of the BFS vial 110, providing external machine-type threads (not shown) directly on the neck 112 may not be a viable option for it would result in an imprecise, unreliable, and/or non-water tight coupling (i.e., the threads may be deformable even if they could be properly manufactured to within the desired tolerances) between the BFS vial 110 and, *e.g.,* the connector 130.

The administration member 180 may include a needle or canula for at least one of ID, subcutaneous, intramuscular, and intravenous injection of a combination of the fluid agent from the BFS vial 110 into a patient. The pre-filled BFS ID injection system 100 may be configured such that, *e.g.,* for IDS injections, approximately one and one half millimeters (1.5-mm) of exposure of the administration end of the administration member 180 may be exposed for insertion into the target. For ease of explanation and description, the figures and the description herein generally refer to the administration member 180 as a needle and are described for convenience with respect to an ID injection. However, it should be noted that the injection may be other than ID and/or the administration member 180 may include a nozzle (not shown) configured to control administration of the combined agent to the patient. The nozzle may include a spray nozzle, for example, configured to facilitate dispersion of the combined agent into a spray. Accordingly, a pre-filled BFS ID injection system 100 fitted with a spray nozzle may be particularly useful in the administration of a combined agent into the nasal passage, for example, or other parts of the body that benefit from a spray application (*e.g.,* ear canal, other orifices). The nozzle may be configured to facilitate formation of droplets of the combined fluid agent. Thus, a pre-filled BFS ID injection system 100 including a droplet nozzle may be useful in the administration of a combined agent by way of droplets, such as administration to the eyes, topical administration, and the like. In each of these cases, the pre-filled BFS ID injection system 100 would more appropriately be referred to as a pre-filled BFS "administration" system 100.

As generally understood, the fluid agent or drug may include any type of agent to be injected into a patient (*e.g.,* mammal, either human or non-human) and capable of producing an effect (alone, or in combination with an active ingredient). Accordingly, the agent may include, but is not limited to, a vaccine, a drug, a therapeutic agent, a medicament, a diluent, and/or the like. The fluid in the BFS vial 110 may comprise, for example, the active ingredient of the drug agent or an object that retains, carries, or holds the active ingredient.

The connector 130, the hub 140, the shield 160, and/or the cap 190 may be composed of a medical grade material such as Polylactic Acid (PLA) and/or another thermoplastic elastomer. The connector 130, the hub 140, the shield 160, and/or the cap 190, may be composed of a thermoplastic polymer or other "hard" plastic (*e.g.,* greater than 80 on the Rockwell "R" scale), including, but not limited to, polybenzimidazole, acrylonitrile butadiene styrene (ABS), polystyrene, polyvinyl chloride, or the like. The BFS vial 110 may be formed of one or more polyolefins such as Low-Density PolyEthylene (LDPE), High-Density PolyEthylene (HDPE), and/or PolyPropylene (PP) and/or one or more other thermoplastics such as Thermoplastic PolyUrethane (TPU).

Fewer or more components 110, 112, 114, 116, 120, 122, 124, 126, 130, 130-1, 130-2, 130-3, 132, 132-1, 134, 136, 138, 140, 142, 144, 146, 146-1, 148, 160, 162, 164, 166, 166-1, 180, 190 and/or various configurations of the depicted components 110, 112, 114, 116, 120, 122, 124, 126, 130, 130-1, 130-2, 130-3, 132, 132-1, 134, 136, 138, 140, 142, 144, 146, 146-1, 148, 160, 162, 164, 166, 166-1, 180, 190 may be included in the pre-filled BFS ID injection system 100. The components 110, 112, 114, 116, 120, 122, 124, 126, 130, 130-1, 130-2, 130-3, 132, 132-1, 134, 136, 138, 140, 142, 144, 146, 146-1, 148, 160, 162, 164, 166, 166-1, 180, 190 may be similar in configuration and/or functionality to similarly named and/or numbered components as described herein. The pre-filled BFS ID injection system 100 (and/or portions thereof) may comprise a disposable, auto-disabled, single-dose delivery assembly operable to be utilized to execute, conduct, and/or facilitate various methods.

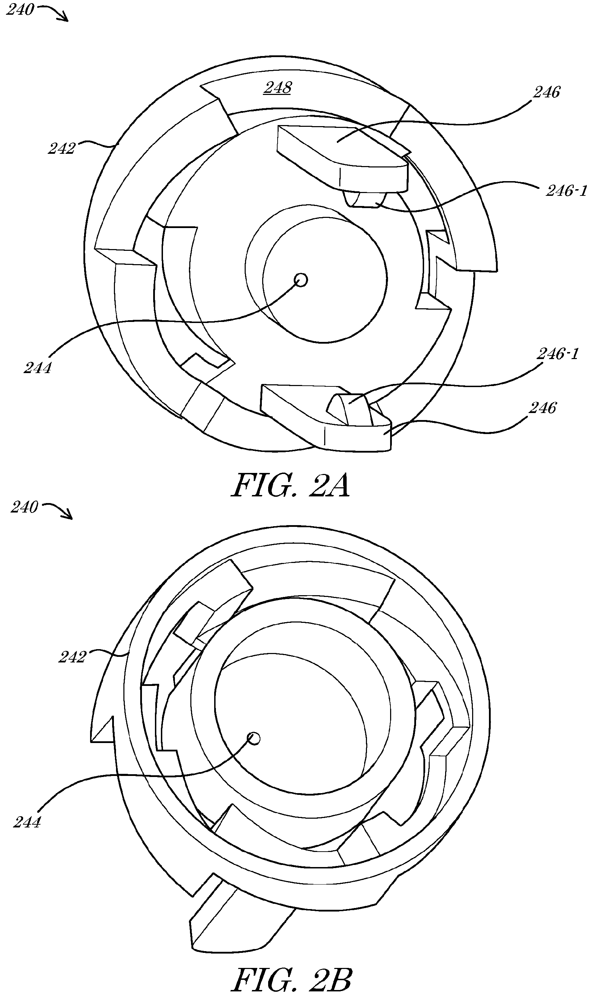

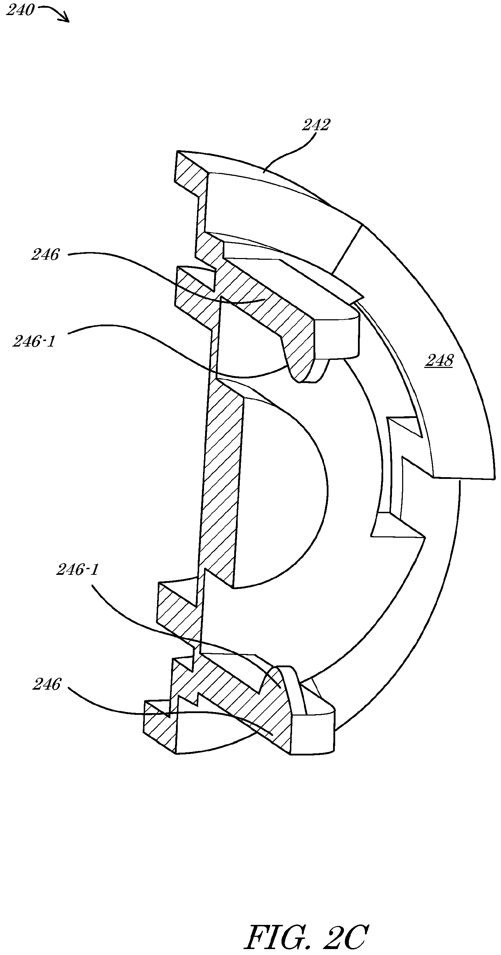

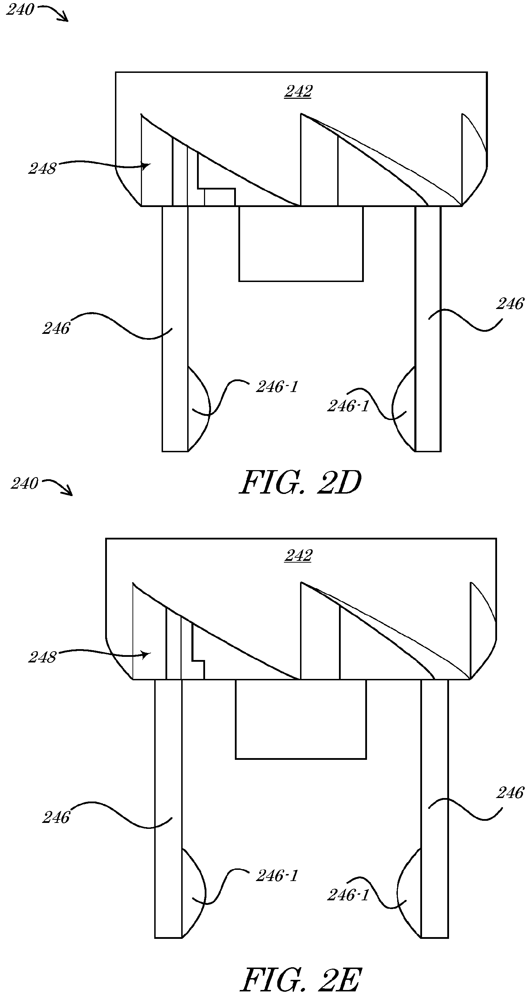

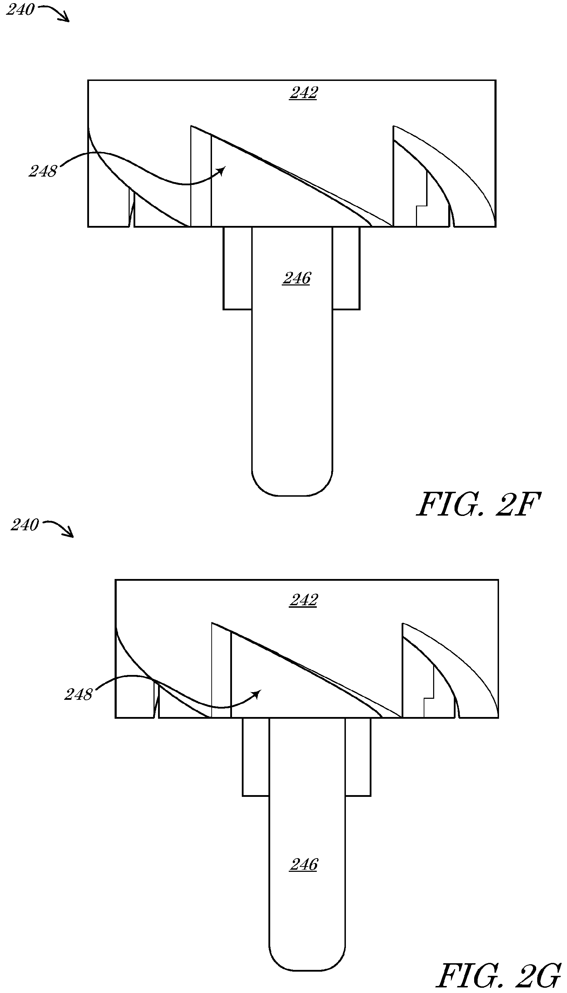

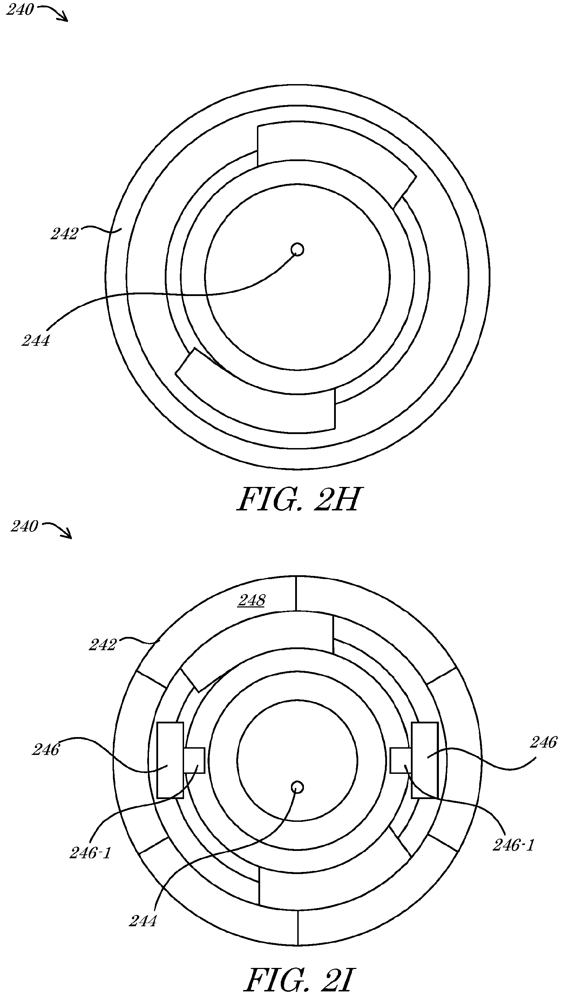

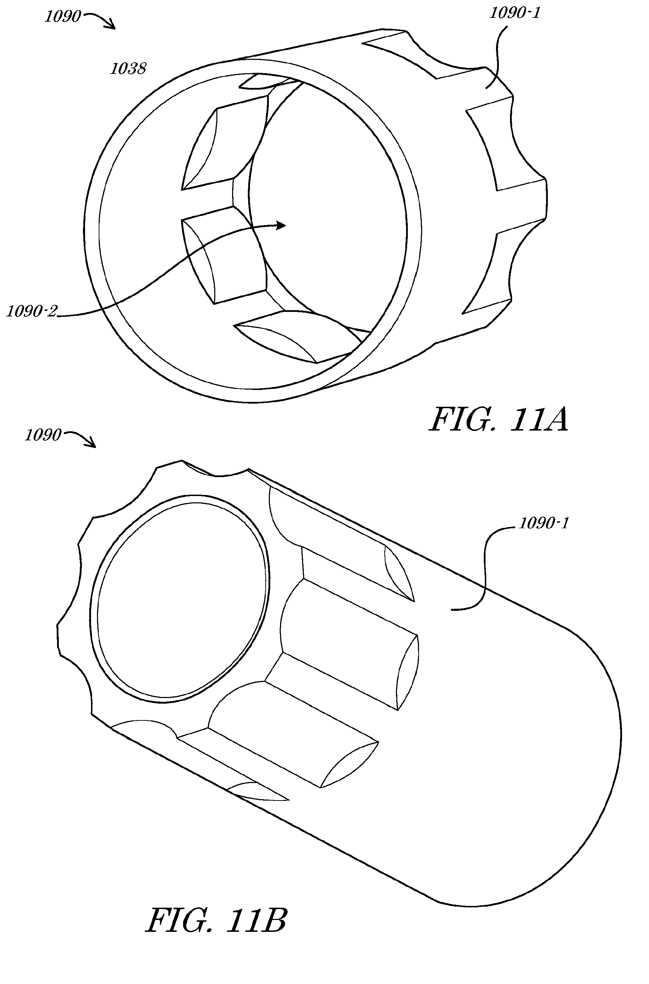

Turning to FIG. 2A, FIG. 2B, FIG. 2C, FIG. 2D, FIG. 2E, FIG. 2F, FIG. 2G, FIG. 2H, and FIG. 2I, front perspective, rear perspective, front perspective cross-section, front, back, left, right, top, and bottom views of an ID injection hub 240 are shown. The ID injection hub 240 may comprise similar features and/or configurations and/or may be similar to the hub 140 of FIG. 1A, FIG. 1B, FIG. 1C, FIG. 1D, FIG. 1E, FIG. 1F, FIG. 1G, FIG. 1H, FIG. 11, FIG. 1J, FIG. 1K, FIG. 1L, and FIG. 1M herein. The ID injection hub 240 may comprise and/or define, for example, a circular, annular, and/or cylindrical base 242. An administration seat 244 may extend through the cylindrical base 242 (and/or through a center portion thereof; not separately labeled) from a first side to a second side thereof. As depicted the administration seat 244 may be eccentric to a central axis of the ID injection hub 240, e.g., to facilitate and/or enable auto-disable functionality as described herein. The ID injection hub 240 may comprise one or more legs 246 extending axially away from the cylindrical base 242 (e.g., in a first axial direction). The legs 246 may comprise pliable and/or elastic or semi-elastic elongated members that extend from the cylindrical base 242 from respective locations radially disposed between the center of the cylindrical base 242 and the radial extents of the cylindrical base 242. Each leg 246 may comprise and/or define a foot 246-1 disposed on a radially interior side of the respective legs 246. The feet 246-1 may comprise, for example, radially inwardly projections disposed proximate to the axial extents of the respective legs 246. The cylindrical base 242 may comprise and/or define one or more cam features or rails 248 disposed radially about the cylindrical base 242. As depicted, for example, the rails 248 may comprise one or more circumferential features such as detents, projections, and/or other shaped portions. The rails 248 may comprise sloped, tapered, cammed, and/or otherwise disjointed and/or uneven surface features, *e.g.,* operable to rotate the hub 240 in the case that the rails 248 are axially engaged with a corresponding track (not shown).

Fewer or more components 242, 244, 246, 246-1, 248 and/or various configurations of the depicted components 242, 244, 246, 246-1, 248 may be included in the ID injection hub 240. The components 242, 244, 246, 246-1, 248 may be similar in configuration and/or functionality to similarly named and/or numbered components as described herein. The ID injection hub 240 (and/or portions thereof) may comprise a disposable, auto-disabled, single-dose delivery assembly operable to be utilized to execute, conduct, and/or facilitate various methods.

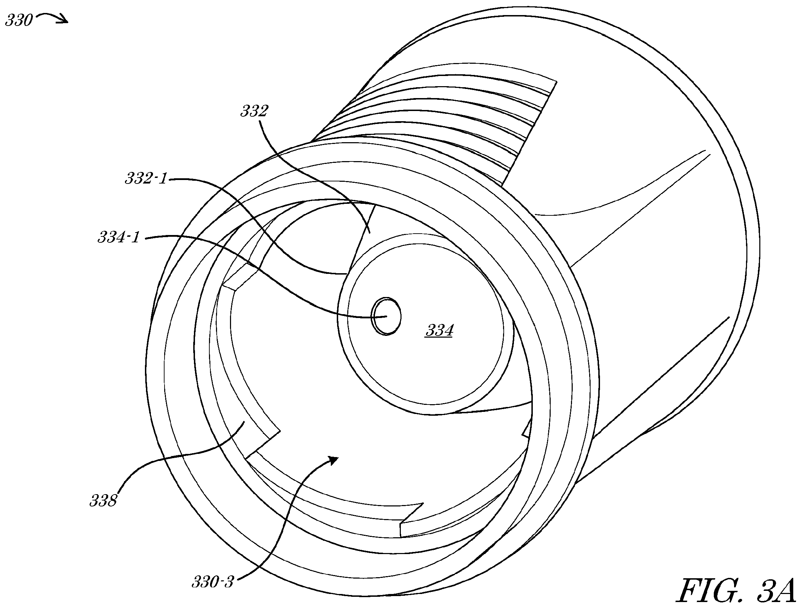

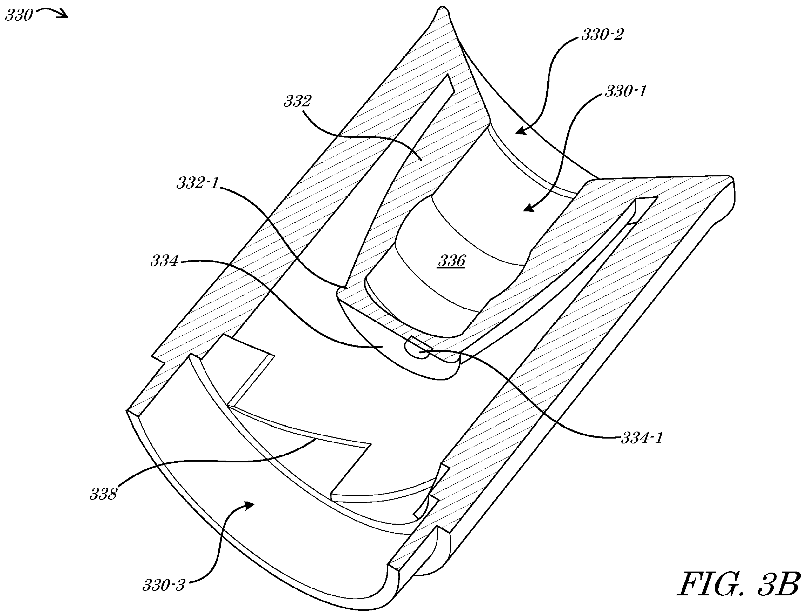

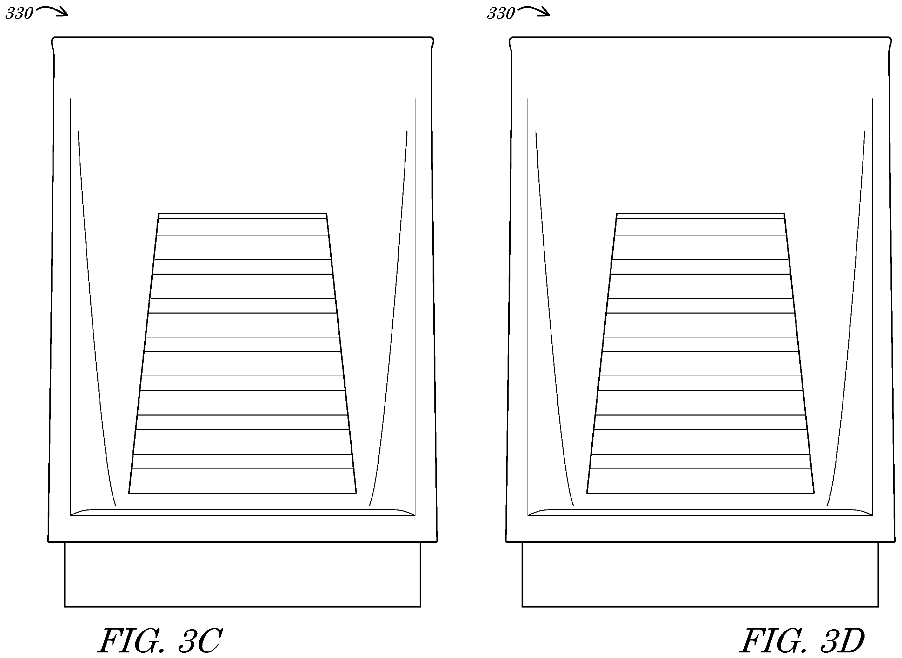

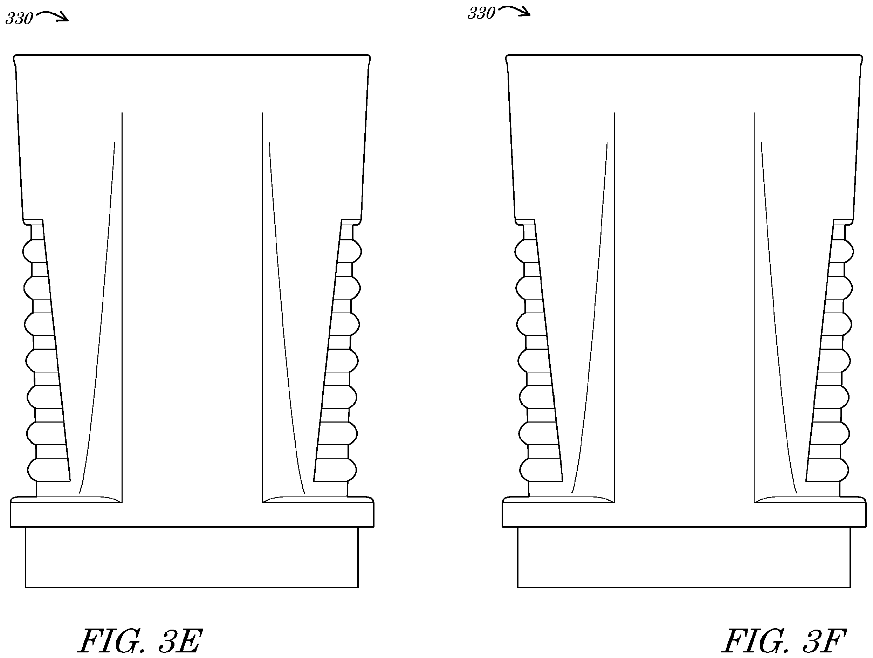

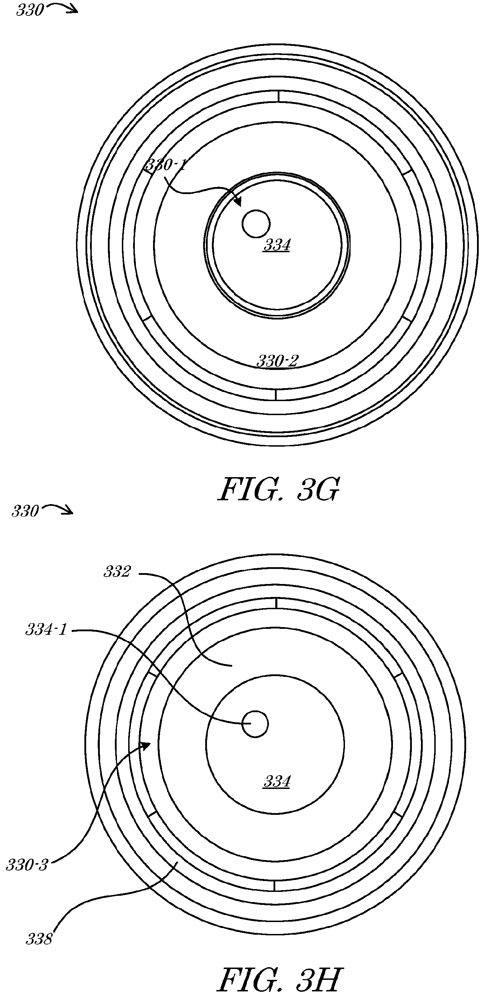

Referring additionally to FIG. 3A, FIG. 3B, FIG. 3C, FIG. 3D, FIG. 3E, FIG. 3F, FIG. 3G, and FIG. 3H, front perspective, perspective cross-section, front, back, left, right, top, and bottom views of a BFS ID injection connector 330. The BFS ID injection connector 330 may comprise similar features and/or configurations and/or may be similar to the connector 130 of FIG. 1A, FIG. 1B, FIG. 1C, FIG. 1D, FIG. 1E, FIG. 1F, FIG. 1G, FIG. 1H, FIG. 11, FIG. 1J, FIG. 1K, FIG. 1L, and FIG. 1M herein. The BFS ID injection connector 330 may comprise, for example, a generally cylindrical body defining a BFS chamber 330-1, a BFS detent 330-2, and/or a hub bore 330-3. A pathway into the BFS chamber 330-1 may be modified from a simply circular (or other chosen geometry) cross-section to provide for easier entry of an inserted mounting flange of a BFS vial (not shown). The hub bore 330-3 may also or alternatively be shaped and/or sized to receive and/or engage with various components (not shown) such as a hub and/or a shield. As depicted for example, the hub bore 330-3 may comprise and/or define an interior cone 332 (*e.g.,* a frustoconical shape) comprising and/or defining a cone detent, exterior channel, and/or lip 332-1. An axial terminus of the interior cone 332 may comprise and/or define an interior surface 334. The interior surface 334 may comprise a planar surface oriented normally to the longitudinal axis of the BFS ID injection connector 330. One or more portions of the interior surface 334 may comprise and/or define a puncture surface 334-1. As depicted in FIG. 3A, for example, the puncture surface 334-1 may comprise a circular or cylindrical portion of the interior surface 334 that comprises an axial thickness that is less than an axial thickness of the remainder of the interior surface 334. In such a manner, for example, a needle or other puncturing member (not shown) may be limited to successfully puncturing the interior surface 334 through the puncture surface 334-1, while engagement with other portions of the interior surface 334 would frustrate puncturing. As depicted, the puncture surface 334-1 may be eccentrically situated such that it is offset from a central portion or axis of the BFS ID injection connector 330. In such a manner, for example, a needle and/or other puncture member that is itself eccentric to the central axis must be rotationally aligned with the puncture surface 334-1 to permit puncturing/breaching of the interior surface 334 (and attendant puncturing and/or engagement with a BFS vial (not shown) seated in the BFS chamber 330-1).

The BFS ID injection connector 330 and/or the BFS chamber 330-1 thereof may comprise an internal groove or seat 336 that is cooperatively sized and configured to receive a mounting flange of a BFS vial (neither shown; e.g., the mounting flange/feature 116 of the BFS vial 110 of FIG. 1A, FIG. 1B, FIG. 1C, FIG. 1D, FIG. 1E, FIG. 1F, FIG. 1G, FIG. 1H, FIG. 11, FIG. 1J, FIG. 1K, FIG. 1L, and FIG. 1M herein). In such a manner, for example, a BFS vial may be selectively and/or removably coupled to the BFS ID injection connector 330. A cooperatively shaped BFS vial may comprise a neck and/or exterior flange that fit within the BFS chamber 330-1 and/or may comprise a conical and/or frustoconical reservoir portion that seats in the BFS detent 330-2. The BFS ID injection connector 330 may comprise and/or define a track 338, e.g., within the hub bore 330-3. The track 338 may comprise and/or define, for example, a plurality of circumferentially arranged and/or offset projections, grooves, channels, and/or shaped elements that are configured to accept and/or guide one or more corresponding features (not shown) of an object inserted into the hub bore 330-3.

Fewer or more components 330-1, 330-2, 330-3, 332, 332-1, 334, 334-1, 336, 338 and/or various configurations of the depicted components 330-1, 330-2, 330-3, 332, 332-1, 334, 334-1, 336, 338 may be included in the BFS ID injection connector 330. The components 330-1, 330-2, 330-3, 332, 332-1, 334, 334-1, 336, 338 may be similar in configuration and/or functionality to similarly named and/or numbered components as described herein. The BFS ID injection connector 330 may comprise a disposable, auto-disabled, single-dose delivery assembly operable to be utilized to execute, conduct, and/or facilitate various methods.

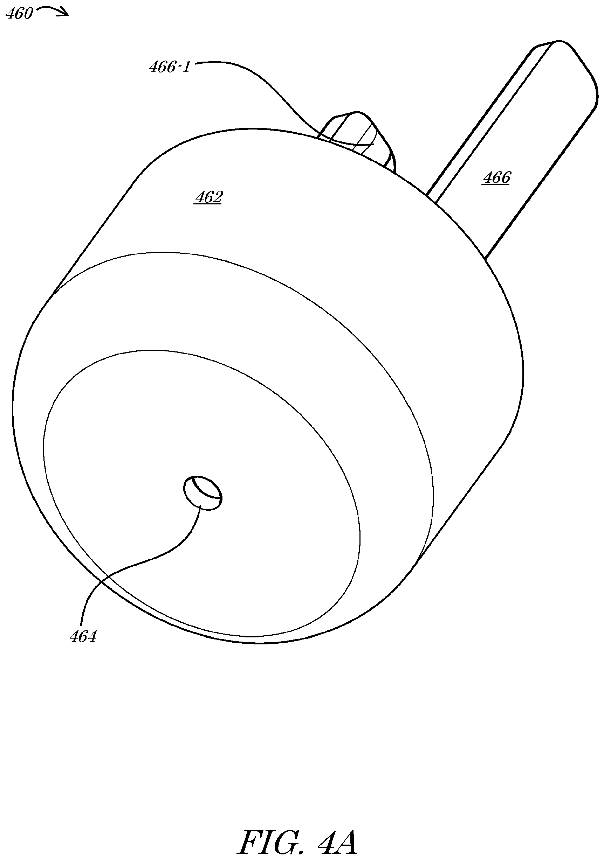

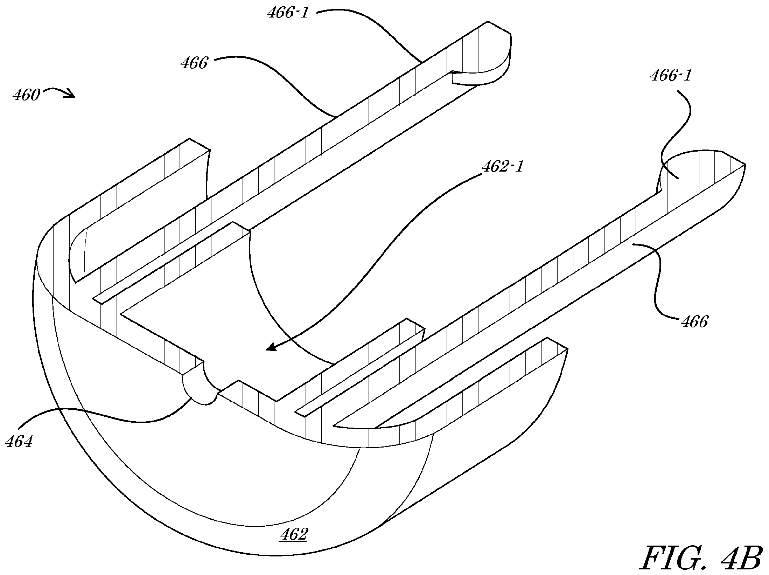

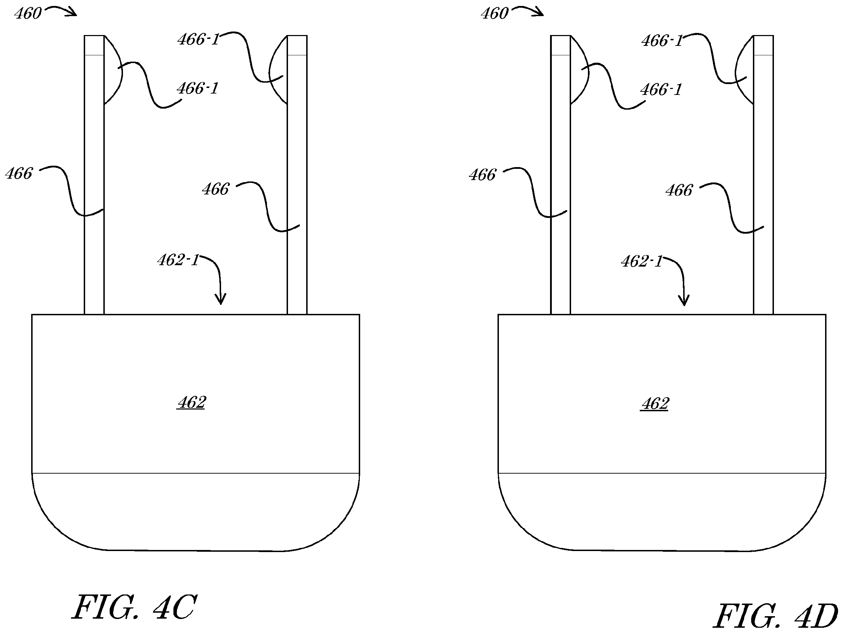

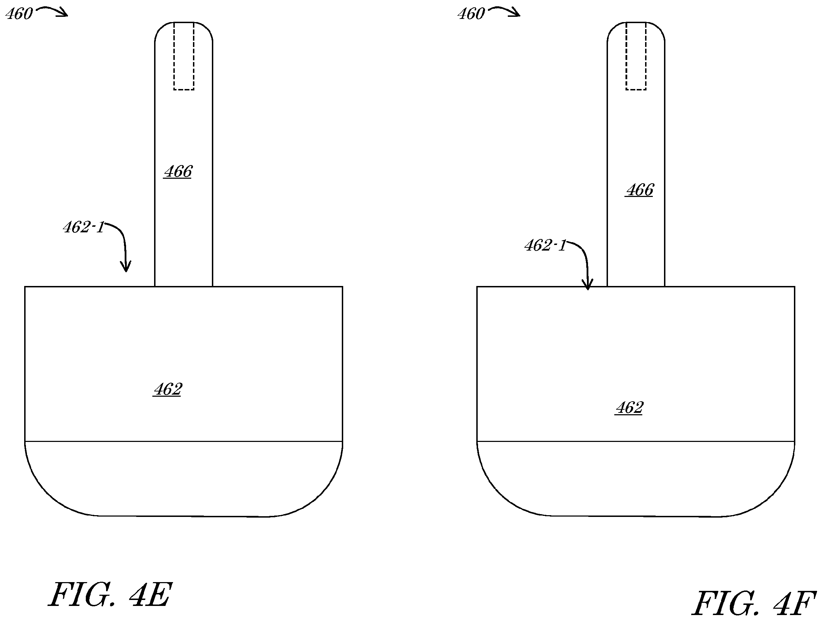

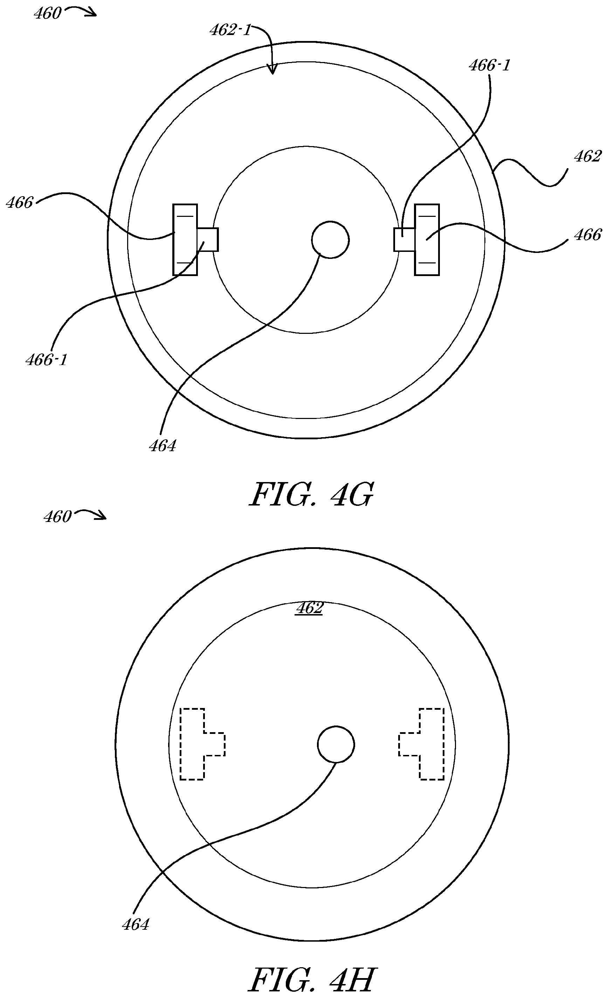

Turning now to FIG. 4A, FIG. 4B, FIG. 4C, FIG. 4D, FIG. 4E, FIG. 4F, FIG. 4G, and FIG. 4H, front perspective, perspective cross-section, front, back, left, right, top, and bottom views of an ID injection shield 460 are shown. The ID injection shield 460 may comprise similar features and/or configurations and/or may be similar to the shield 160 of FIG. 1A, FIG. 1B, FIG. 1C, FIG. 1D, FIG. 1E, FIG. 1F, FIG. 1G, FIG. 1H, FIG. 1I, FIG. 1J, FIG. 1K, FIG. 1L, and FIG. 1M herein. The ID injection shield 460 may comprise and/or define, for example, a circular, annular, and/or cylindrical body 462 defining an interior volume 462-1. An administration bore 464 may extend through the cylindrical body 462 (and/or through a center portion thereof; not separately labeled) from a first side to a second side thereof. As depicted, the administration bore 464 may be eccentric to a central axis of the ID injection shield 460, *e.g.,* to facilitate and/or enable auto-disable functionality as described herein. The ID injection shield 460 may comprise one or more legs 466 extending axially away from the cylindrical body 462 (*e.g.,* in a first axial direction). The legs 466 may comprise pliable and/or elastic or semi-elastic elongated members that extend from the cylindrical body 462 from respective locations radially disposed between the center of the cylindrical body 462 and the radial extents of the cylindrical body 462. Each leg 466 may comprise and/or define a foot 466-1 disposed on a radially interior side of the respective legs 466. The feet 466-1 may comprise, for example, radially inwardly projections disposed proximate to the axial extents of the respective legs 466.

Fewer or more components 462, 462-1, 464, 466, 466-1 and/or various configurations of the depicted components 462, 462-1, 464, 466, 466-1 may be included in the ID injection shield 460. The components 462, 462-1, 464, 466, 466-1 may be similar in configuration and/or functionality to similarly named and/or numbered components as described herein. The ID injection shield 460 may comprise a disposable, auto-disabled, single-dose delivery assembly operable to be utilized to execute, conduct, and/or facilitate various methods.

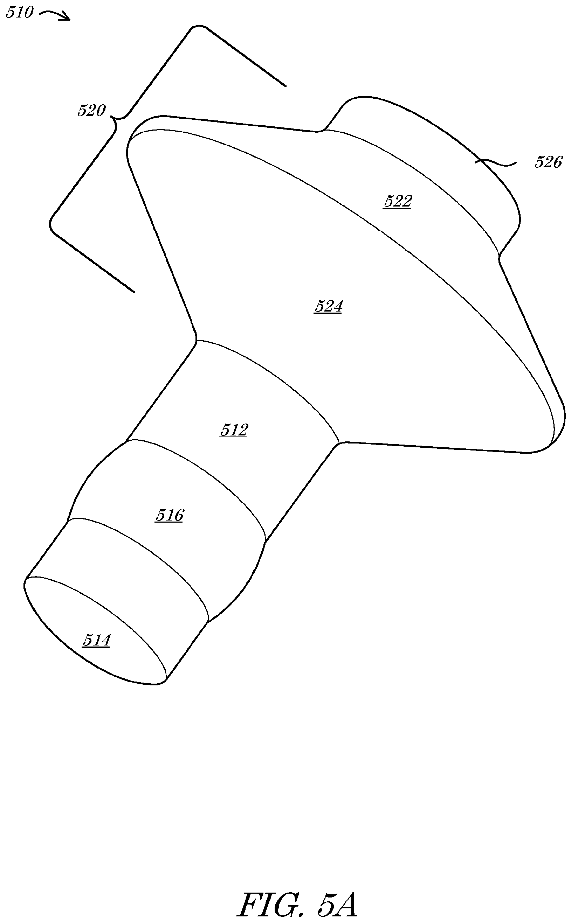

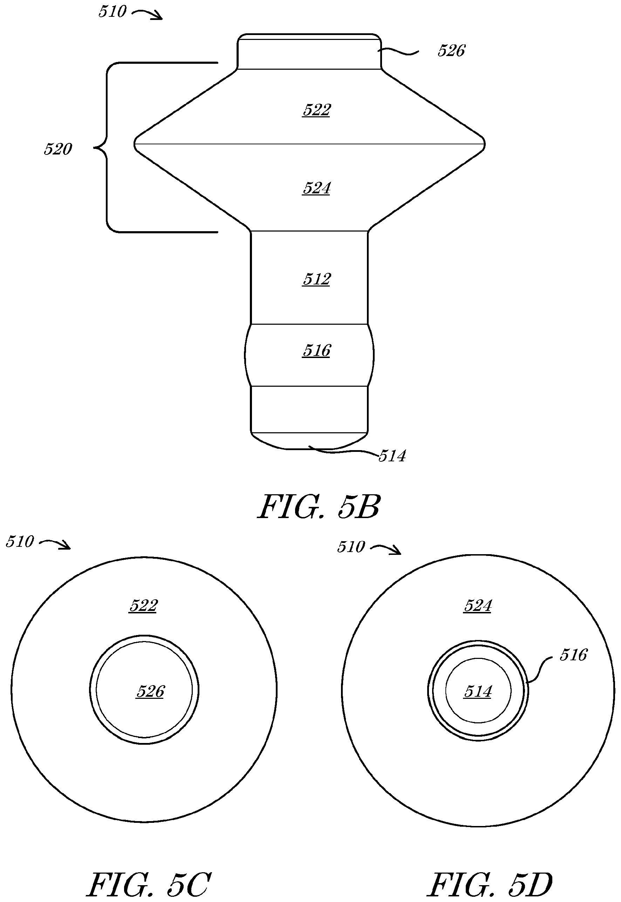

Referring additionally to FIG. 5A, FIG. 5B, FIG. 5C, and FIG. 5D, front perspective, front, top, and bottom views of pre-filled BFS vial 510. Pre-filled BFS vial 510 may comprise similar features and/or configurations and/or may be similar to the BFS vial 110 of FIG. 1A, FIG. 1B, FIG. 1C, FIG. 1D, FIG. 1E, FIG. 1F, FIG. 1G, FIG. 1H, FIG. 11, FIG. 1J, FIG. 1K, FIG. 1L, and FIG. 1M herein. The pre-filled BFS vial 510 may comprise and/or define, for example, a vial neck 512, a fluid seal 514, a mounting flange 516, and/or a collapsible reservoir 520 (*e.g.,* comprising an upper reservoir surface 522, a lower reservoir surface 524, and/or a push surface 526). The neck 512 may comprise a cylindrical and longitudinally extended feature that is sealed at a proximal end by the fluid seal 514, comprises the mounting flange 516 disposed on an exterior surface and along the length thereof, and/or is coupled to and/or in communication with the collapsible reservoir 520 at a distal end. The collapsible reservoir 520 may comprise an (*e.g.,* one or more) ellipsoid, disc, conical, and/or frustoconical shape or volume defining the upper reservoir surface 522 and the lower reservoir surface 524). As depicted, for example, the collapsible reservoir 520 may comprise a shape configured such that the upper reservoir surface 522 may be collapsed into or onto the lower reservoir surface 524, thereby substantially collapsing the collapsible reservoir 520. In the case that the fluid seal 514 is punctured (as described herein), application of axial compressive force to the push surface 526 and/or the upper reservoir surface 522 may cause the collapsible reservoir 520 to fold inward upon itself, thereby expelling a volume of fluid that was previously housed within the collapsible reservoir 520. In such a manner, for example, the collapsible reservoir 520 may be compressed to expel a vaccine and/or other medicament from the BFS vial 510 (e.g., and into a patient; not shown). The collapsing may not be readily reversible. The push surface 526 may not comprise a large enough grip surface, for example, to permit human gripping for application of reverse or axially outward expansive force.

Fewer or more components 512, 514, 516, 520, 522, 524, 526 and/or various configurations of the depicted components 512, 514, 516, 520, 522, 524, 526 may be included in the pre-filled BFS vial 510. The components 512, 514, 516, 520, 522, 524, 526 may be similar in configuration and/or functionality to similarly named and/or numbered components as described herein. The pre-filled BFS vial 510 may comprise a disposable, auto-disabled, single-dose delivery assembly (or portion thereof) operable to be utilized to execute, conduct, and/or facilitate various methods.

### III. Pre-filled BFS ID Medical Agent Delivery Systems - Seven (7) Component Embodiment

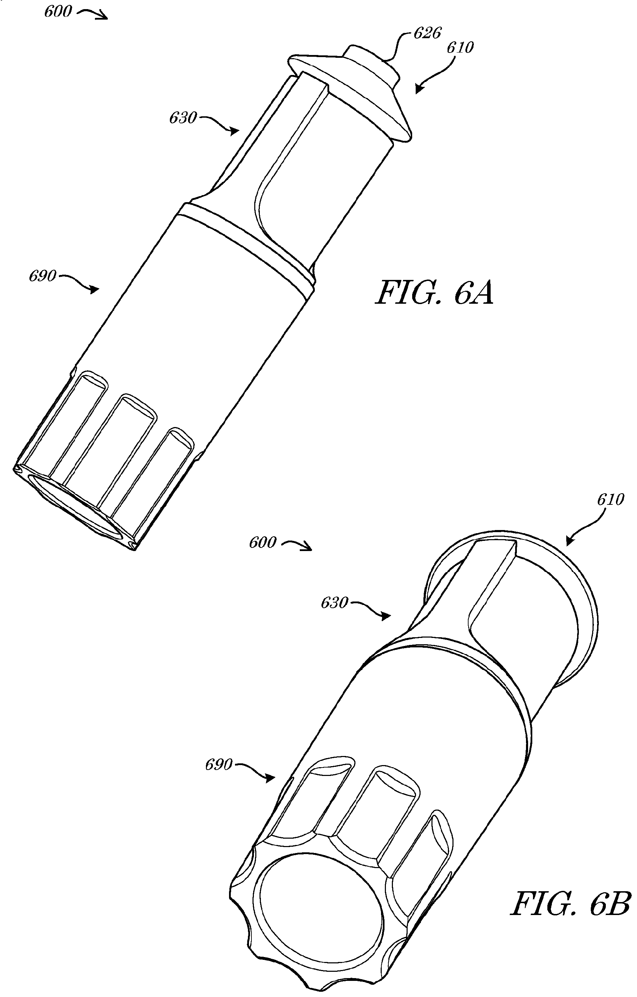

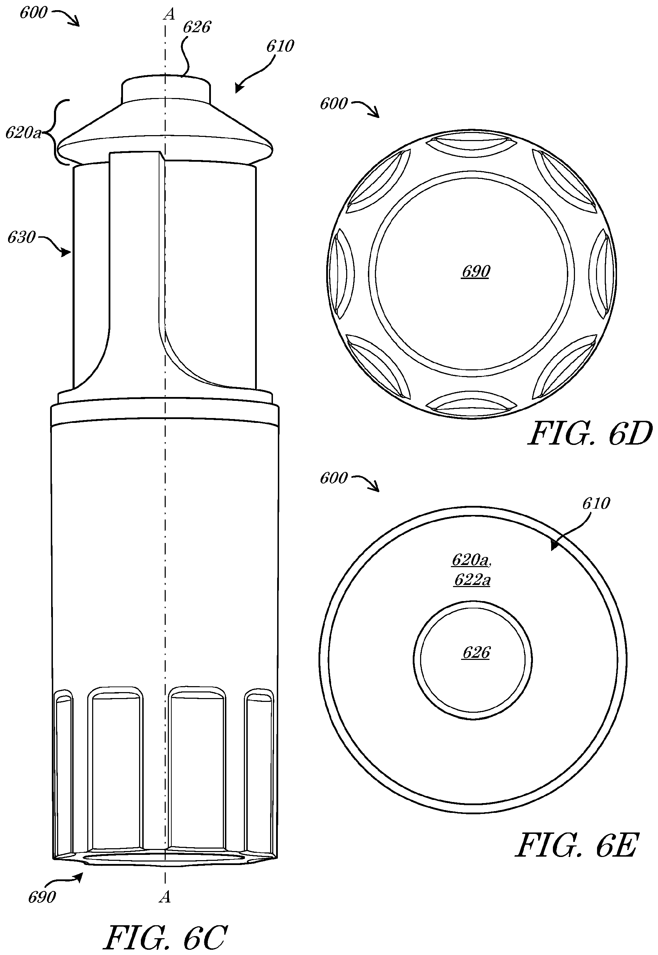

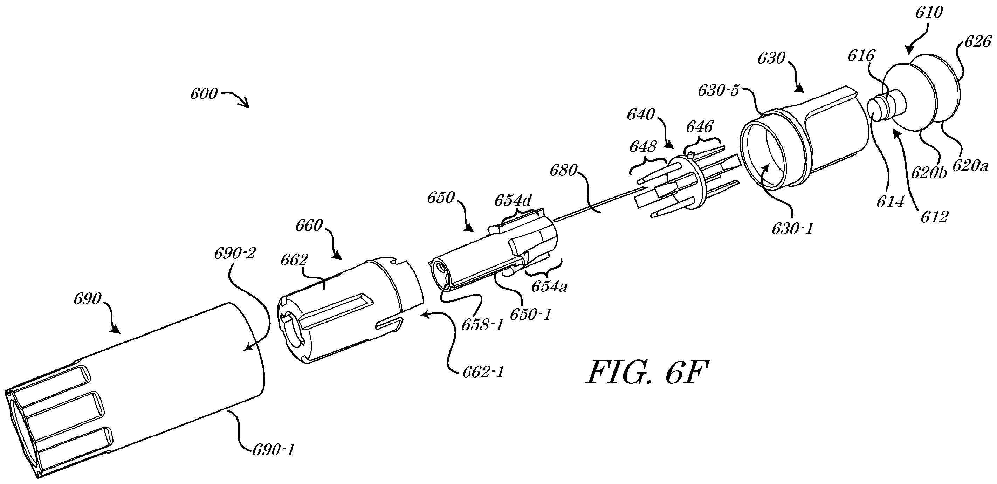

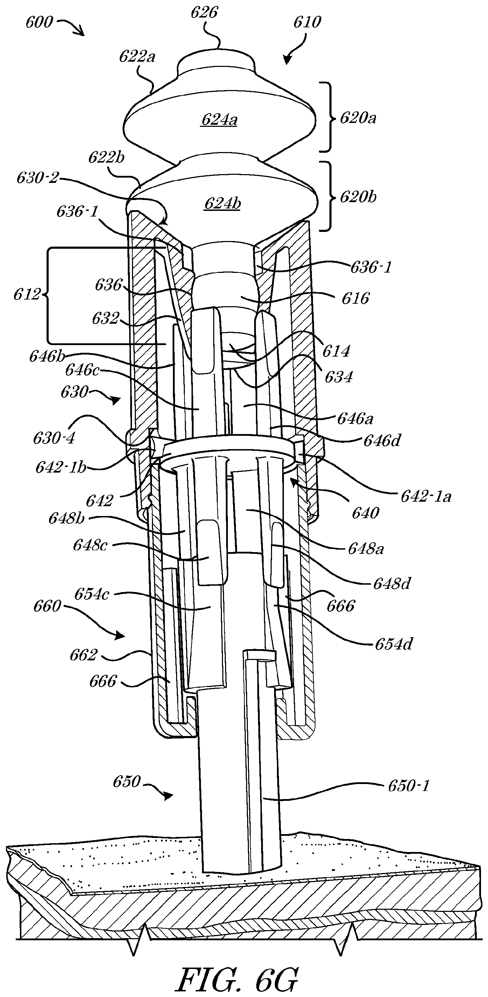

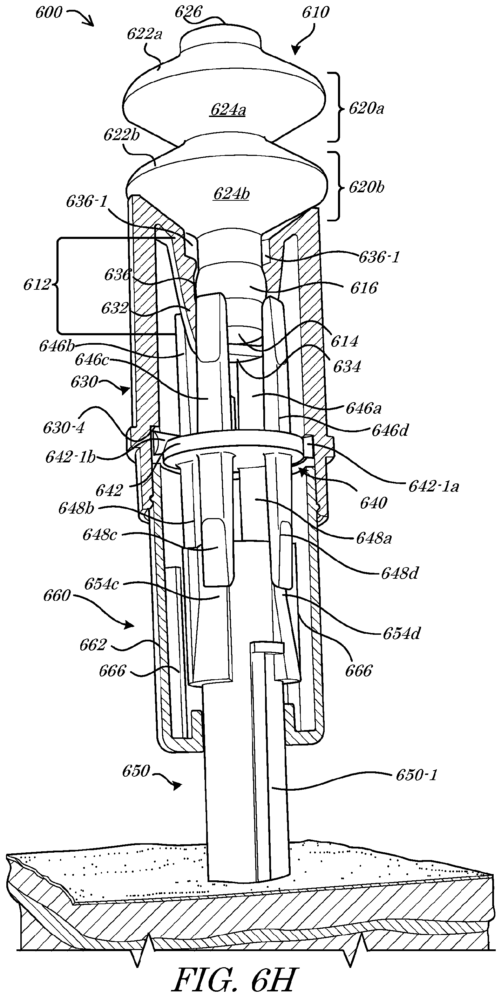

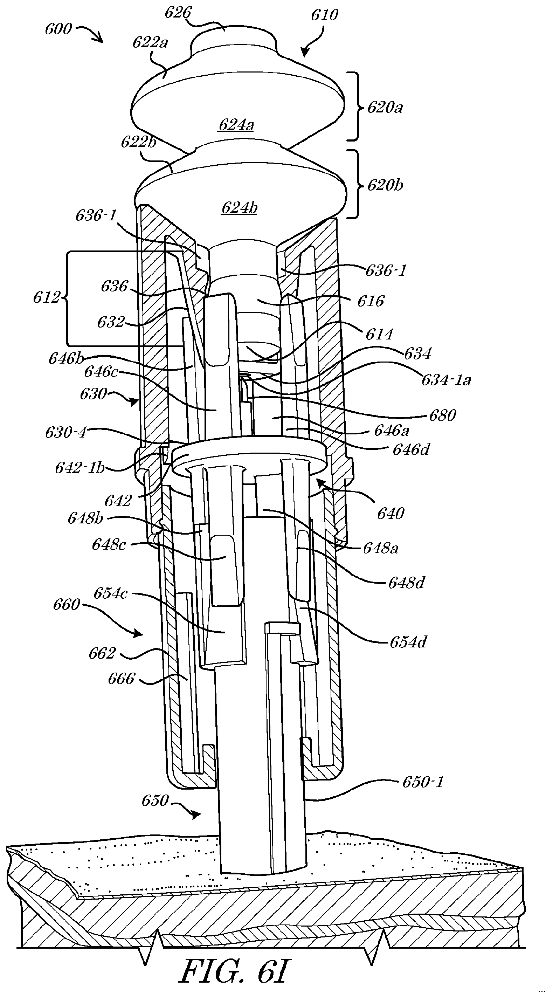

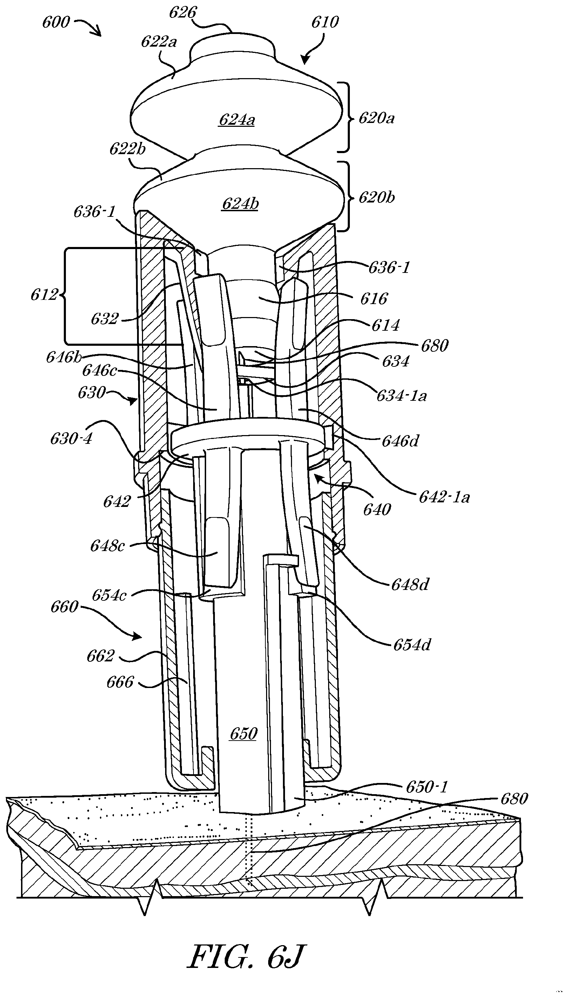

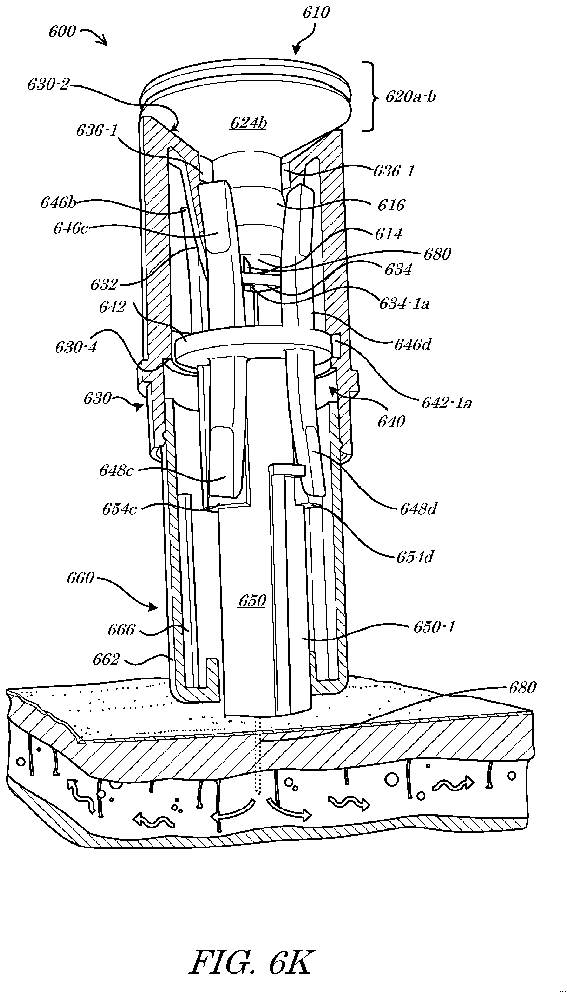

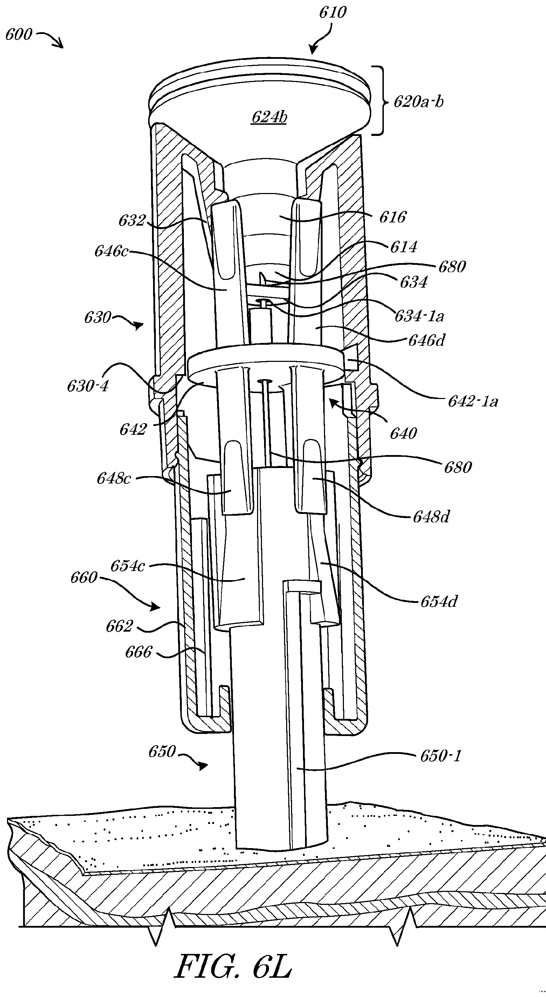

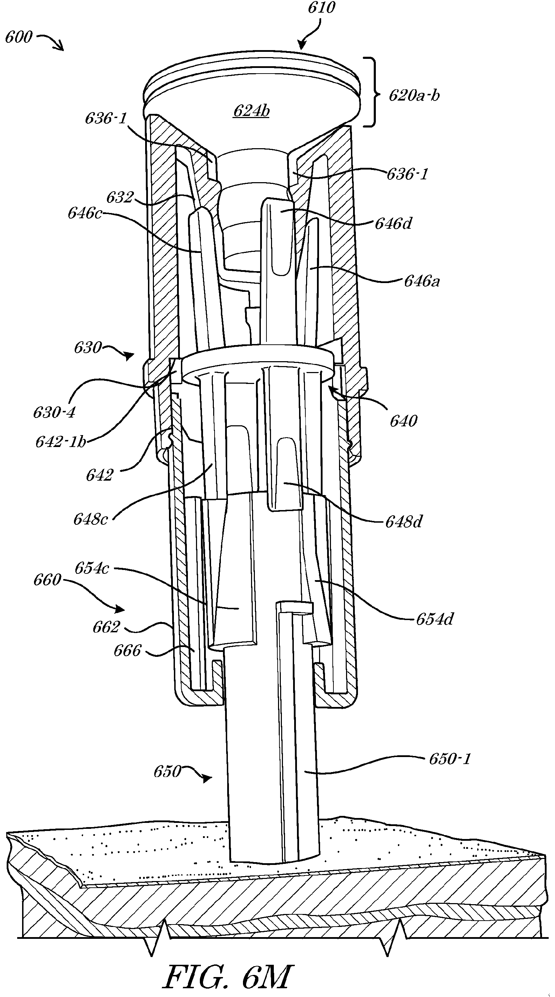

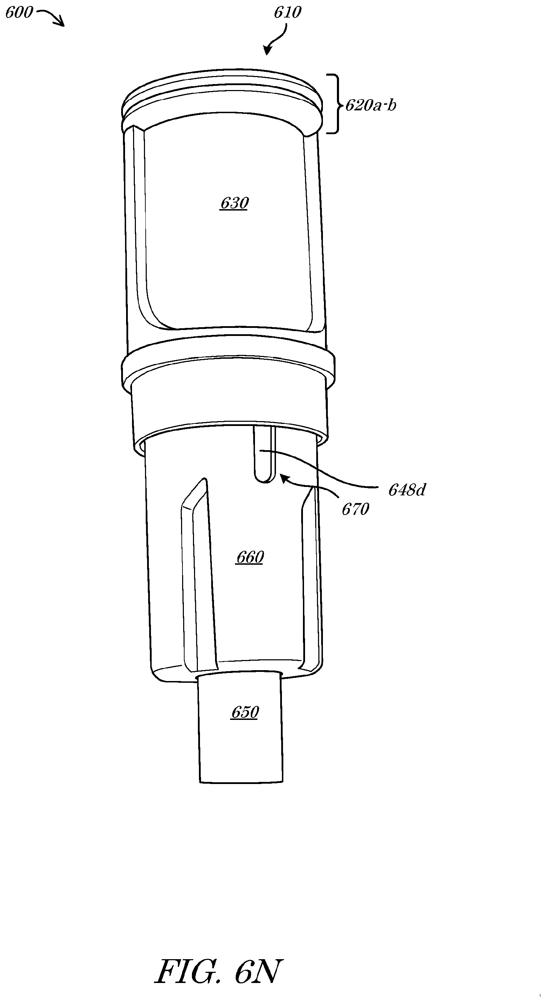

Referring initially to FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N, various views of a pre-filled BFS ID injection system 600 according to some embodiments are shown. In some embodiments, the pre-filled BFS ID injection system 600 may comprise various inter-connected and/or modular components such as a BFS vial 610 (or bottle, unit, ampule, etc.) comprising and/or defining a vial neck 612, a fluid seal 614, a mounting flange 616, and/or one or more collapsible reservoirs 620a-b (*e.g.,* comprising an upper reservoir surface 622a-b, a lower reservoir surface 624a-b, and/or a push surface 626). According to some embodiments, the BFS vial 610 may comprise a plastic and/or synthetic vial that is constructed via any practicable manufacturing techniques. In some embodiments the BFS manufacturing technique may be utilized. In embodiments where the BFS vial 610 is constructed utilizing BFS manufacturing techniques, special characteristics may necessarily be imparted to the BFS vial 610 due to the BFS manufacturing technique and may accordingly be inherent in the BFS vial 610. Such characteristics may include, but are not limited to (and indeed may not require), the BFS vial 610 (i) being manufactured in an aseptic and/or sterile environment, (ii) being automatically filled and sealed in the aseptic/sterile environment, (iii) being comprised of a soft plastic, *e.g.,* having a Shore/Durometer "D" hardness of between 60 and 70, (iv) being formed in two conjoined halves along an axial seam, (v) being geometrically limited to symmetrically moldable features, and/or (vi) being limited to formation in particular temperature ranges (*e.g.,* above freezing). While a particular shape and configuration of the BFS vial 610 is depicted for ease of illustration and explanation, other shapes and/or configurations may be utilized without deviating from some embodiments. The BFS vial 610 may not include, in some embodiments for example, the mounting flange 616 and/or may comprise a side or seam flange (not shown; e.g., formed between different co-manufactured units of BFS vials 610 and providing and/or defining a cut or punch surface at which the different units are separated post-manufacture).

According to some embodiments, the pre-filled BFS ID injection system 600 may comprise a mounting collar or connector 630 in which the BFS vial 610 is seated. The connector 630 may comprise and/or define, for example, a BFS chamber 630-1, a BFS detent 630-2, a hub bore 630-3, an interior flange 630-4, an exterior flange 630-5, an interior cone 632, an interior surface 634, one or more puncture surfaces 634-1a, 634-1b, a seat 636, and/or a flange seat 636-1. In some embodiments, the pre-filled BFS ID injection system 600 may comprise a hub 640 comprising an annular, circular, and/or cylindrical base 642, one or more base guides 642-1a, 642-1b, an administration seat 644, one or more upper legs 646, and/or one or more lower legs 648. According to some embodiments, the pre-filled BFS ID injection system 600 may comprise a piston 650 that may selectively engage with the hub 640. The piston 650 may comprise and/or define, for example, a piston body 650-1 (*e.g*., cylindrical as shown) with one or more leg tracks 654a, 654d formed and/or disposed thereupon. The leg tracks 654a, 654d may be configured, for examples, such that a coupling of the piston 650 and the hub 640 engages the one or more lower legs 648 with the leg tracks 654a, 654d. According to some embodiments, engagement of the one or more lower legs 648 with the leg tracks 654a, 654d via application of axial/longitudinal force (*e.g.,* applied and/or received along an axis "A" of the pre-filled BFS ID injection system 600) may urge the lower legs 648 radially outward, thereby generating a radial elastic force.

In some embodiments, the pre-filled BFS ID injection system 600 may comprise an injection stopper or shield 660 comprising a cylindrical body 662 defining an interior volume 662-1, an administration bore 664 disposed at a proximal end of the interior volume 662-1, and/or one or more shoulders 666 disposed and/or formed within the interior volume 662-1. In some embodiments, the injection shield 660 may comprise and/or define one or more axial slots or slits 670 (*e.g.,* on or through an exterior wall of the cylindrical body 662). According to some embodiments, the pre-filled BFS ID injection system 600 may comprise an administration member 680 and/or a cap 690. The administration member 680 may, for example, be coupled to and/or retained by the administration seat 644 of the hub 640 and/or the cap 690 may be seated with the exterior flange 630-5 of the connector 630 (*e.g.,* selectively covering and/or housing portions of the connector 630, the injection shield 660, and/or the piston 650). According to some embodiments, the pre-filled BFS ID injection system 600 may comprise a modular design consisting of separately constructed components 610, 630, 640, 650, 660, 680, 690 cooperatively arranged and coupled to one another. According to some embodiments, some of the components 610, 630, 640, 650, 660, 680, 690 may be manufactured, created, molded, and/or otherwise formed together.

In some embodiments, the collapsible reservoirs 620a-b of the BFS vial 610 may together (in the case there are more than one) define a fluid volume that may be filled (fully or partially) with a fluid or other agent (not separately shown) such as a medicament. According to some embodiments, the fluid may be injected into the BFS vial 610 in a sterile environment during manufacture via a BFS process and sealed within the BFS vial 610 via the fluid seal 614. The fluid seal 614 may comprise a portion of the molded BFS vial 610 for example that is configured to be pierced to expel the fluid, *e.g.,* such as by providing a flat or planar piercing surface, being selectively formed with a particular material thickness, and/or by being oriented normal to an axis of the BFS vial 610 (and/or the pre-filled BFS ID injection system 600). In some embodiments, the fluid seal 614 may comprise a foil, wax, paper, and/or other thin, pierceable object or layer coupled to the BFS vial 610. In some embodiments, the neck 612 of the BFS vial 610 may comprise the mounting flange 616 such as, *e.g.,* the "doughnut"-shaped exterior flange depicted (and/or one or more other tabs, detents, protrusions, and/or other features). In some embodiments, the mounting flange 616 may also or alternatively comprise a side or seam flange (not depicted), *e.g.,* whether disposed/formed on the neck 612 or elsewhere on the BFS vial 610. According to some embodiments, and as depicted, the neck 612 may be cylindrically shaped. In some embodiments, the neck 612 may comprise one or more other cross-sectional shapes or configurations such as a triangle, square, rectangle, pentagon, hexagon, star, and/or octagon shape. In some embodiments, the shape of the neck 612 may correspond to a type of fluid agent stored in the BFS vial 610.

According to some embodiments, the connector 630 may be axially engaged to couple with the BFS vial 610 via application of an axial mating force. The connector 630 (and/or the BFS chamber 630-1 thereof) may be urged onto the neck 612 of the BFS vial 610, for example (or *vice versa*)*,* such that it accepts and/or selectively couples to the mounting flange 616 (*e.g.,* via the seat 636 and/or the flange seat 636-1), thereby removably coupling the BFS vial 610 and the mounting collar 630. According to some embodiments, the BFS chamber 630-1 (and/or the BFS detent 630-2) may be shaped to correspond to and/or cooperatively mate with the shape of the neck 612 and/or of at least one of the collapsible reservoirs 620a-b (*e.g.,* a second or proximal reservoir 620b) of the BFS vial 610. In the case that the neck 612 is cylindrically or triangularly shaped, for example, the BFS chamber 630-1 may comprise a cylindrical or triangular opening and/or passage, respectively. In some embodiments, uncoupling of the BFS vial 610 and the connector 630 may be mechanically prohibited. The mounting flange 616 may effectively lock into the seat 636 (and/or the flange seat 636-1) connector 630 once inserted, for example, preventing or inhibiting removal thereafter. According to some embodiments, when seated in the BFS chamber 630-1 (and/or the BFS detent 630-2), the BFS vial 610 may be substantially shrouded by the connector 630. As depicted, for example, in a seated configuration, only a first or distal reservoir 620a and/or the upper reservoir surface 622a thereof and/or the push surface 626 may be externally exposed and/or accessible. According to some embodiments, a first end of the connector 630 (and/or of the pre-filled BFS ID injection system 600) may be referred to as a distal end, as it is distal from a site of injection (not separately labeled). Utilizing such terminology, the BFS vial 610 may be coupled to and/or seated in the connector 630 at the distal end thereof.

In some embodiments, the hub 640 may be selectively coupled to and/or seated in the hub bore 630-3 of a proximal end of the connector 630. According to some embodiments, the upper legs 646 of the hub 640 may extend into the hub bore 630-3 and engage with the interior cone 632 thereof. Each upper leg 646 may, in some embodiments, comprise a pliable member that is elastically and radially offset as the hub 640 is inserted axially deeper into the hub bore 630-3 such that the upper legs 646 travel along the increasing outer diameter of the interior cone 632. In such a manner, for example, elastic and/or axial resistance may increase as the hub 640 is inserted axially deeper into the hub bore 630-3. According to some embodiments the hub 640 may couple to and/or retain the administration member 680. The administration member 680 may be inserted into the administration seat 644 of the hub 640, for example, such that a first or piercing end (not separately labeled) is disposed within the hub bore 630-3, *e.g.,* in the case that the hub 640 is seated in the hub bore 630-3, and a second or administration end (not separately labeled) extends axially distal from the BFS vial 610. According to some embodiments, in a first state or engagement position, the administration member 680 and/or the piercing end thereof may be positioned adjacent to (*e.g.,* axially adjacent to) the interior surface 634 (and/or a specific portion thereof such as the puncture surface(s) 634-1a, 634-1b). In some embodiments, the administration end and/or a distal portion of the administration member 680 may be housed, shrouded, and/or covered by the piston, 650, the shield 660 and/or the cap 690 *(*e.g., in the case that the latter has been installed and/or has not yet been removed).

According to some embodiments, the pre-filled BFS ID injection system 600 may be assembled and/or provided in the first state or engagement position where the cap 690 is coupled to the connector 630 and the administration member 680 is completely or substantially aligned (*e.g.,* axially) with a guide hole 658-1 at a proximal end of the piston. In some embodiments, in the first state or engagement position the base guides 642-1a, 642-1b may comprise radially protruding tabs or features that are coupled to and/or held by the injection shield 660 in a first radial orientation. According to some embodiments, in the first state or engagement position the upper legs 646 of the hub 640 may be engaged to a first amount or degree with the interior cone 632 of the connector 630 and the lower legs 648 may be engaged to a first amount or degree with the leg tracks 654a, 654d of the piston 650.

In some embodiments, the cap 690 may be removed, exposing the proximal end of the piston 650 (and, *e.g.,* the proximal portions of the injection shield 660). In the first state or engagement position, the administration end of the administration member 680 may be shrouded by the piston 650, *e.g.,* to prevent accidental injury once the cap 690 is removed. According to some embodiments, a user (*e.g.,* self-administration patient, doctor, nurse, etc.) may transition the pre-filled BFS ID injection system 600 from the first state or engagement position to a second state or engagement position whereby the BFS vial 610 is pierced and the fluid (or a portion thereof, such as a liquid portion, while a gas portion remains) therein is injected into the target area. As depicted in FIG. 6G, for example, the proximal end of the piston 650 may be placed normal to the target surface and pushed against the target surface. As depicted in FIG. 6H, initial axial movement (in response to applied axial force) may cause the distal end of the piston 650 to advance with respect to the lower legs 648 (*e.g.,* a third lower leg 648c and a fourth lower leg 648d). The lower legs 648 may, for example, engage with or further engage with respective leg tracks 654c-d of the piston 650. According to some embodiments, the shoulders 666 may limit or prevent radial deflection/bending of the lower legs 648 and thereby cause the piston 650 and the hub 640 (and the administration member 680) to move together. This may, for example, cause the administration member 680 to remain shrouded within the piston 650 despite the overall axial compression of the pre-filled BFS ID injection system 600.

In some embodiments, as continued axial force or pressure is applied, the lower legs 648 may be forced to flex radially outward by the shape of the respectively engaged leg tracks 654c-d, thereby creating or enhancing an elastic and/or radial force and increasing the axial resistance between the hub 640 and the piston 650. The increased resistance may, in some embodiments, become equal to or greater than a resistance between the upper legs 646 and the interior cone 632, thereby causing the upper legs 646 (and the hub 640 and the piston 650) to advance with respect to the upper cone 632, *e.g.,* as depicted in FIG. 6I. According to some embodiments, as the base guides 642-1a, 642-1b advance axially toward the interior cone 632 they may encounter and/or engage with respective internal tracks (not shown) within the hub bore 630-3. In some embodiments, the base guides 642-1a, 642-1b may be beveled such that as they advance axially (*e.g.,* upward as shown) from the first state or engagement position, the bevel engages with the track, thereby causing the hub 640 to rotate with respect to the connector 630. In some embodiment, such rotation may be approximately three degrees (3°). In some embodiments, such a rotation may cause the administration member 680 and/or the piercing end thereof to attain a piercing position with respect to the interior surface 634 (and/or one or more puncture surfaces 634-1a, 634-1b thereof). As depicted, for example, the administration member 680 (and the corresponding administration seat 644) may be axially offset (*e.g.,* eccentrically positioned) from the central axis "A" of the pre-filled BFS ID injection system 600. In such embodiments, rotation of the hub 640 may cause the piercing end of the administration member 680 to travel along a circular path over or on the interior surface 634. In the case that a particular portion of the interior surface 634 is thinner and/or otherwise configured to accept the administration member 680, such as one or more of the puncture surfaces 634-1a, 634-1b, the administration member 680 may pierce the interior surface 634 and travel into and pierce the fluid seal 614 of the BFS vial 610 upon achievement of a particular axial insertion distance. According to some embodiments, the pre-filled BFS ID injection system 600 may be configured such that a rotation in the range of three degrees (3°) to seven degrees (7°) of the hub 640 may cause the administration member 680 to become aligned for piercing the BFS vial 610.

As depicted in FIG. 6J, the second state or engagement position may be achieved once sufficient axial force has been applied to engage the upper legs 646 with the interior cone 632 to an extent where the administration member pierces the BFS vial 610 and the lower legs 648, freed from the shoulders 666 due to axial movement away from the injection shield 660, are able to radially deflect and fully engage with the leg tracks 654c, 654d such that the lower extents of the lower legs 648 engage with and are stopped by the stop tabs 656 of the piston 650. The advancement/engagement may also cause the administration end of the administration member 680 to become aligned with and advance through the guide hole 658-1 and into the target. According to some embodiments, once the administration member 680 has entered the target, the push surface 626 of the BFS vial 610 may be axially forced toward the injection site, causing the collapsible reservoir(s) 620a-b to compress against the BFS detent 630-2 (as depicted in FIG. 6K) and thereby causing any or all fluid therein to advance through the administration member 680 and into the target *(*e.g., forming an ID "bleb"; not shown). In some embodiments, the BFS detent 630-2 acting as a compression surface for the collapsible reservoir(s) 620a-b to compress against may be provided by a different configuration of a connector 630 coupled to the BFS vial 610. The different/alternate configuration of the connector 630 may, for example, not comprise an ID injector and/or may not comprise or utilize the hub 640, the piston 650, and/or the shield 660. According to some embodiments, the different/alternate configuration of the connector 630 may comprise a snap-on (or other mating style) injection hub (not shown) that mates the administration member 680 with the BFS vial 610 and provides the BFS detent 630-2 acting as a compression surface for the collapsible reservoir(s) 620a-b to compress against.

In some embodiments, the pre-filled BFS ID injection system 600 may be advanced (*e.g.,* automatically) to a third state or engagement position upon and/or in response to a release of the axial pressure/force from the piston 650. Removal or decreasing the axial force or pressure may cause the elastic (*e.g.,* spring) force of the legs 646, 648 to release, thereby causing the upper legs 646 to retreat from the interior cone 632 and/or the lower legs 648 to retreat from the respective leg tracks 654c, 654d (as depicted in FIG. 6L). According to some embodiments, the reverse axial travel or retreating of the legs 646, 646 may cause the base guides 642-1a, 642-1b to exit the interior tracks of the connector 630, *e.g.,* in their shifted radial positions, and engage with a distal edge or perimeter of the injection shield 660 at an engagement location that differs from the original engagement or seat location of the base guides 642-1a, 642-1b with respect to the injection shield 660. According to some embodiments, such engagement may cause the base guides 642-1a, 642-1b to rotate the hub 640 with respect to the injection shield 660 and the piston 650. In some embodiments, the rotation of the hub 640 may cause the administration member 680 to become radially offset by approximately forty-five degrees (45°), *e.g.,* thereby misaligning the administration member with the puncture surfaces 634-1a, 634-1b and/or with the guide hole 658-1.

The rotation may also or alternatively cause the lower legs 648c-d to fall off of or disengage with the leg tracks 654c-d such that further attempts to axially engage the pre-filled BFS ID injection system 600 may fail to bend the lower legs 648 outward, *e.g.,* rendering the pre-filled BFS ID injection system 600 inoperable in the third state or engagement position (as depicted in FIG. 6M). In some embodiments, the rotation may also or alternatively cause the axially-offset administration member 680 to become misaligned from the hole made by the piercing of the BFS vial 610 and/or from the guide hole 658-1. In such a manner, for example, the pre-filled BFS ID injection system 600 may become deactivated and/or rendered unusable (*e.g.,* for reuse). In some embodiments, these rotational misalignments and/or the collapsing of the collapsible reservoir(s) 620a-b may selectively and automatically disable the pre-filled BFS ID injection system 600 after use. According to some embodiments, an automatic visual indication of the third state or engagement position may be provided via the axial slits 670. Upon attaining the third state or engagement position due at least in part to and/or resulting in the rotation of the hub 640 inside of the pre-filled BFS ID injection system 600 for example, one of the lower legs 648 such as the fourth lower leg 648d depicted in FIG. 6N may become visible due to the achieved rotational alignment of the hub 640 subsequent to the administration/injection. In the case that the pre-filled BFS ID injection system 600 is in the first and/or second state or engagement position, no lower leg 648 may be visible through the axial slots 670. In the case that the lower legs 648 are provided in a highly visible color such as red, a user may readily identify the used/third state of the pre-filled BFS ID injection system 600 by simply viewing the fourth lower leg 648d through the axial slit 670.

According to some embodiments, hub 640, piston 650, and shield 660 combination may be utilized to couple and/or mate the administration member 680 with the connector 630. In some embodiments, the connector 630 may provide a mechanism via which the administration member 680 may be coupled to be in fluid communication with a soft plastic BFS vial 610 in a reliable manner. Due to the nature of the BFS plastic and/or process and/or the small form-factor of the BFS vial 610, in some embodiments for example, providing external machine-type threads (not shown) directly on the neck 612 may not be a viable option for it may result in an imprecise, unreliable, and/or non-water tight coupling (*i.e.,* the threads may be deformable even if they could be properly manufactured to within the desired tolerances) between the BFS vial 610 and, *e.g.,* the connector 630.

In some embodiments, the administration member 680 may include a needle or canula for at least one of ID, subcutaneous, intramuscular, and intravenous injection of a combination of the fluid agent from the BFS vial 610 into the target/patient. According to some embodiments, the pre-filled BFS ID injection system 600 may be configured such that, *e.g.,* for ID injections, approximately one and one half millimeters (1.5-mm) of exposure of the administration end of the administration member 680 may be exposed for insertion into the target. In some embodiments, the pre-filled BFS ID injection system 600 may be configured such that, *e.g.,* for subcutaneous injections, approximately eight millimeters (8-mm) of exposure of the administration end of the administration member 680 may be exposed for insertion into the target.

For ease of explanation and description, the figures and the description herein generally refer to the administration member 680 as a needle and are described for convenience with respect to an ID injection. However, it should be noted that, in other embodiments, the injection may be other than ID and/or the administration member 680 may include a nozzle (not shown) configured to control administration of the combined agent to the patient. The nozzle may include a spray nozzle, for example, configured to facilitate dispersion of the combined agent into a spray. Accordingly, a pre-filled BFS ID injection system 600 fitted with a spray nozzle may be particularly useful in the administration of a combined agent into the nasal passage, for example, or other parts of the body that benefit from a spray application (*e.g.,* ear canal, other orifices). In other embodiments, the nozzle may be configured to facilitate formation of droplets of the combined fluid agent. Thus, a pre-filled BFS ID injection system 600 including a droplet nozzle may be useful in the administration of a combined agent by way of droplets, such as administration to the eyes, topical administration, and the like. In each of these cases, the pre-filled BFS ID injection system 600 would more appropriately be referred to as a pre-filled BFS "administration" system 600.

As generally understood, the fluid agent or drug may include any type of agent to be injected into a patient (*e.g.,* mammal, either human or non-human) and capable of producing an effect (alone, or in combination with an active ingredient). Accordingly, the agent may include, but is not limited to, a vaccine, a drug, a therapeutic agent, a medicament, a diluent, and/or the like. In some embodiments, the fluid in the BFS vial 610 may comprise, for example, the active ingredient of the drug agent or an object that retains, carries, or holds the active ingredient.

According to some embodiments, the connector 630, the hub 640, the piston 650, the shield 660, and/or the cap 690 may be composed of a medical grade material such as Polylactic Acid (PLA) and/or another thermoplastic elastomer. In some embodiments, the connector 630, the hub 640, the piston 650, the shield 660, and/or the cap 690, may be composed of a thermoplastic polymer or other "hard" plastic (*e.g.,* greater than 80 on the Rockwell "R" scale), including, but not limited to, polybenzimidazole, acrylonitrile butadiene styrene (ABS), polystyrene, polyvinyl chloride, or the like. In some embodiments, the BFS vial 610 may be formed of one or more polyolefins such as Low-Density PolyEthylene (LDPE), High-Density PolyEthylene (HDPE), and/or PolyPropylene (PP) and/or one or more other thermoplastics such as Thermoplastic PolyUrethane (TPU).

In some embodiments, fewer or more components 610, 612, 614, 616, 620a-b, 622a-b, 624a-b, 626, 630, 630-1, 630-2, 630-3, 630-4, 630-5, 632, 634, 634-1a, 634-1b, 636, 636-1, 640, 642, 642-1a, 642-1b, 644, 646, 646a-d, 648, 648a-d, 650, 650-1, 654a, 654c-d, 656, 658-1, 660, 662, 662-1, 664, 666, 670, 680, 690, 690-1, 690-2 and/or various configurations of the depicted components 610, 612, 614, 616, 620a-b, 622a-b, 624a-b, 626, 630, 630-1, 630-2, 630-3, 630-4, 630-5, 632, 634, 634-1a, 634-1b, 636, 636-1, 640, 642, 642-1a, 642-1b, 644, 646, 646a-d, 648, 648a-d, 650, 650-1, 654a, 654c-d, 656, 658-1, 660, 662, 662-1, 664, 666, 670, 680, 690, 690-1, 690-2 may be included in the pre-filled BFS ID injection system 600 without deviating from the scope of embodiments described herein. In some embodiments, the components 610, 612, 614, 616, 620a-b, 622a-b, 624a-b, 626, 630, 630-1, 630-2, 630-3, 630-4, 630-5, 632, 634, 634-1a, 634-1b, 636, 636-1, 640, 642, 642-1a, 642-1b, 644, 646, 646a-d, 648, 648a-d, 650, 650-1, 654a, 654c-d, 656, 658-1, 660, 662, 662-1, 664, 666, 670, 680, 690, 690-1, 690-2 may be similar in configuration and/or functionality to similarly named and/or numbered components as described herein. In some embodiments, the pre-filled BFS ID injection system 600 (and/or portions thereof) may comprise a disposable, auto-disabled, single-dose delivery assembly (or portion thereof) operable to be utilized to execute, conduct, and/or facilitate various methods such as one or more BFS ID injection methods that utilize internal elastic forces to automatically enable and/or disable an injection system based on application of axial force thereto.

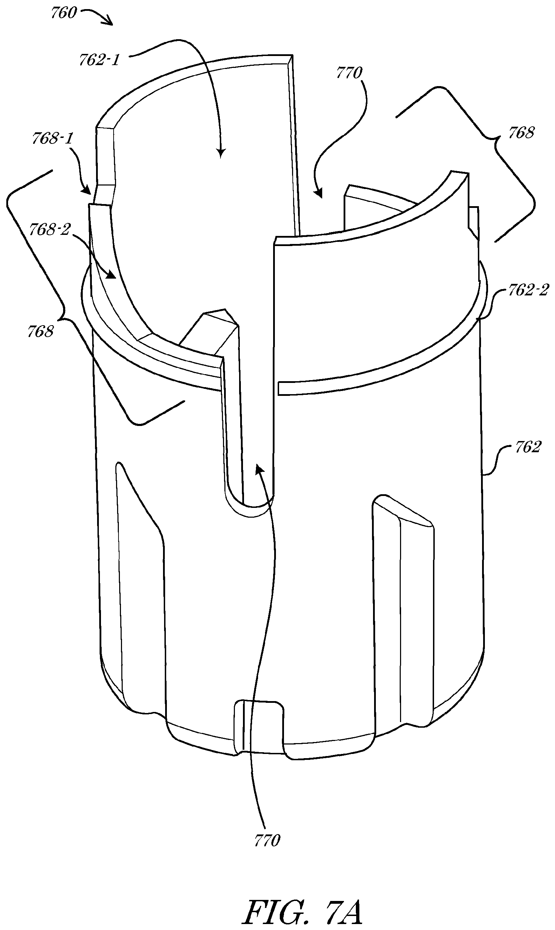

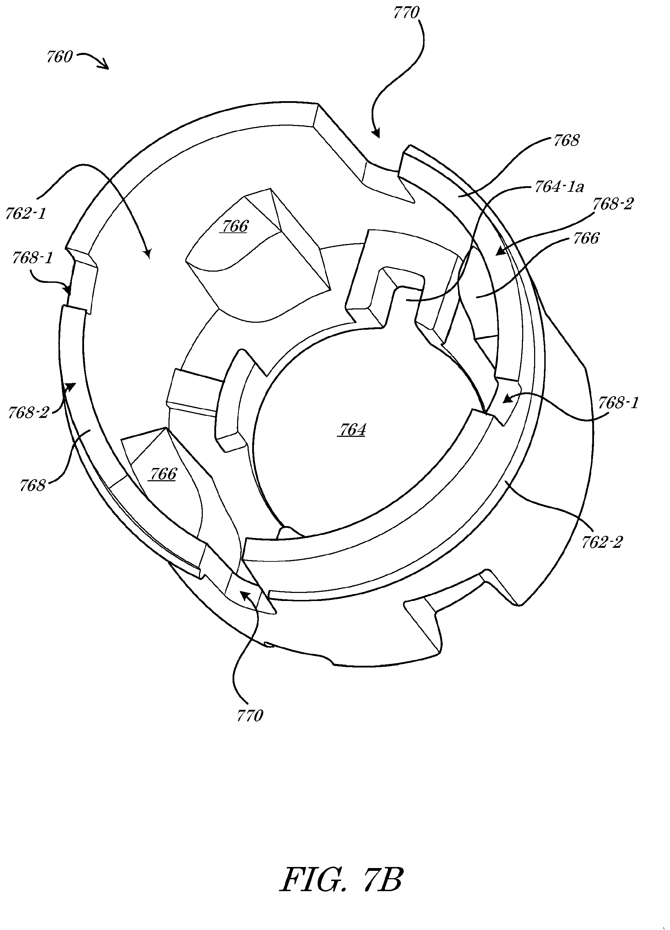

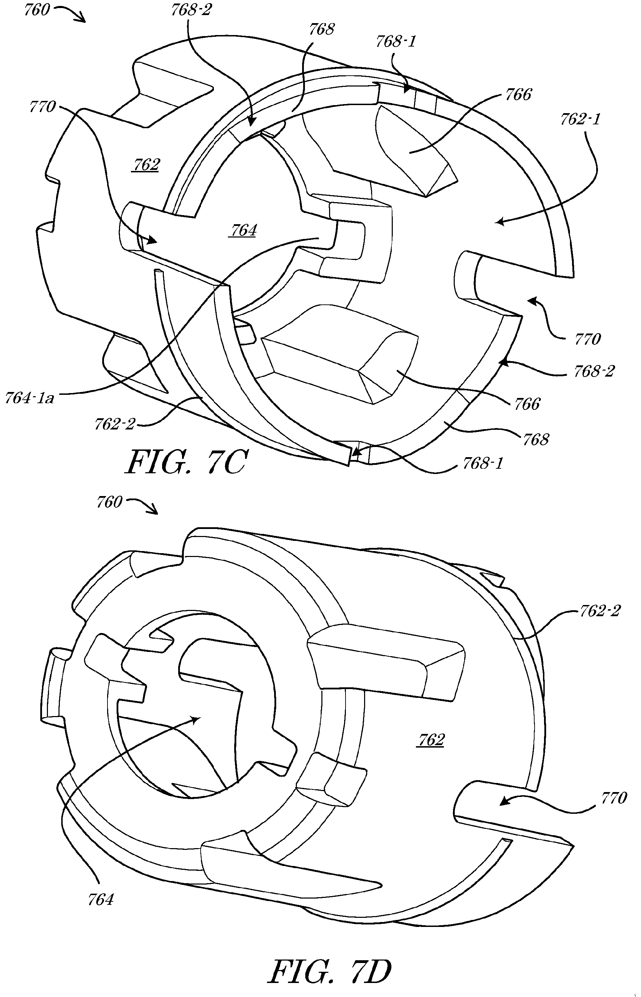

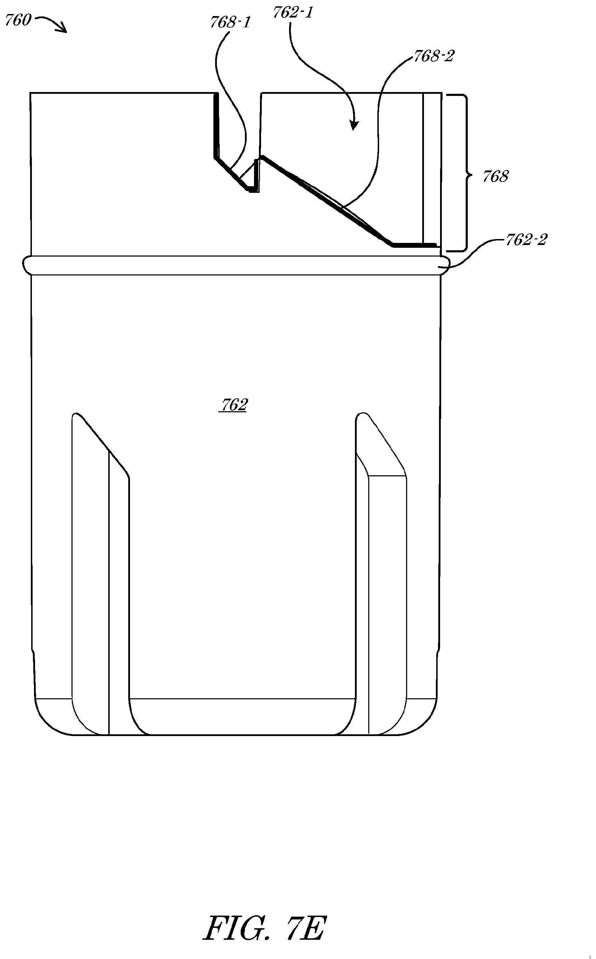

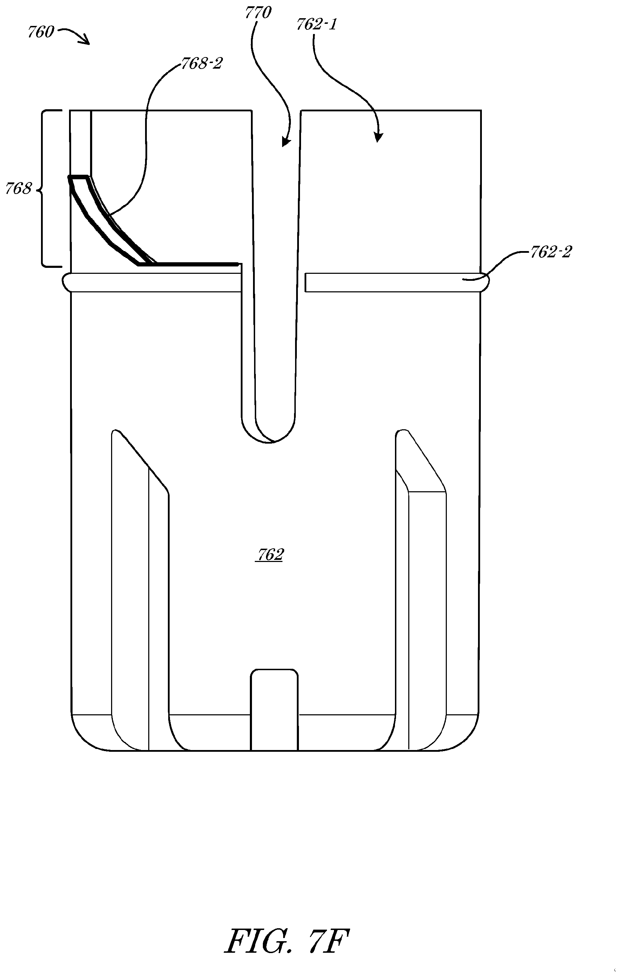

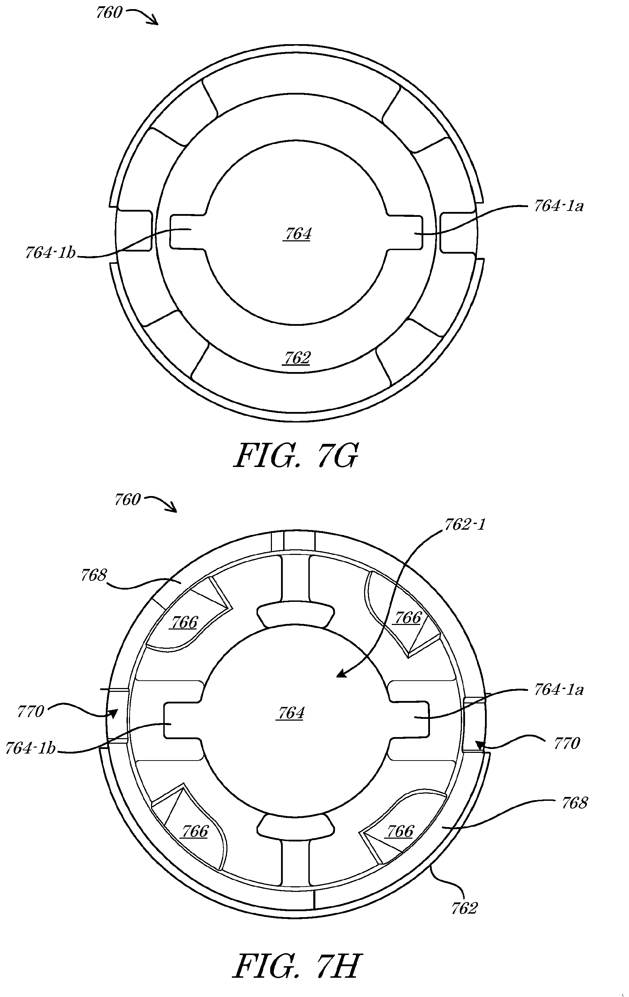

Turning now to FIG. 7A, FIG. 7B, FIG. 7C, FIG. 7D, FIG. 7E, FIG. 7F, FIG. 7G, and FIG. 7H, front left perspective, top left perspective, top right perspective, bottom left perspective, front, left, top, and bottom views of an ID injection shield 760 according to some embodiments are shown. In some embodiments, the ID injection shield 760 may comprise similar features and/or configurations and/or may be similar to the injection shield 660 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein. The ID injection shield 760 may comprise and/or define, for example, a circular, annular, and/or cylindrical body 762 defining an interior volume 762-1. In some embodiments, an outer rounded flange or rib 762-2 may be disposed around the cylindrical body 762 near a distal/upper end of the ID injection shield 760. The outer rib 762-2 may, for example, be selectively and cooperatively mated with a groove (not shown) of a connector component (not shown; *e.g.,* the connector 630 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). According to some embodiments, the ID injection shield 760 may comprise and/or define a piston bore 764 at a proximal end thereof. The piston bore 764 may be sized to accommodate, for example, the passing of a body of a piston (not shown; *e.g.,* the cylindrical body 662 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). In some embodiments, such as in the case that the piston comprises side or axial flanges (not shown), the ID injection shield 760 and/or the piston bore 764 may comprise and/or define corresponding flange guides 764-1a, 764-1b, *e.g.,* that may act as anti-rotation devices with respect to the piston and the ID injection shield 760.

According to some embodiments, the interior volume 762-1 of the ID injection shield 760 may comprise and/or define a plurality of protrusions or shoulders 766 that are sized, spaced, and/or shaped to guide and/or limit radial movement/bending of elastic legs (not shown; e.g., the lower legs 648 of the hub 640 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). In some embodiments, the ID injection shield 760 may comprise and/or define a circumferential track 768. The circumferential track 768 may, for example, comprise a distal perimeter or end surface of the cylindrical body 762 that is shaped to form a particular profile. As well depicted in FIG. 7A, FIG. 7B, FIG. 7C, and FIG. 7E, for example, while certain portions of the cylindrical body 762 at the distal end may extend to a maximum or full cylindrical extent (axially), other portions may comprise and/or be formed or cut to reduced heights and/or profiles. In some embodiments, two (2) symmetrical circumferential tracks 768 may be formed on opposite sides of the ID injection shield 760. According to some embodiments, the circumferential track(s) 768 may define and/or comprise a first or start portion 768-1 and/or a second or rotation portion 768-2. The start portion 768-1 may comprise, for example, a trap or catch profile that is shaped and sized to hold or retain a base guide/tab of a hub component (not shown; *e.g.,* the base guides 642-1a, 642-1b of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). Any base guide/tab engaged to sit within the start portion 768-1 (*e.g.,* at or during a first state or engagement position) may, for example, only be free to move from the start portion 768-1 in a single axial (*e.g.,* upward as depicted) direction and may be restrained from rotational movement by the start portion 768-1. In some embodiments, the rotation portion 768-2 may comprise a sloped portion of the perimeter where, in the case that a base guide/tab engages axially (*e.g.,* downward as depicted) toward the perimeter, the base guide/tab may be urged to slide along the sloped rotation portion 768-2 thereby causing a rotation of the base guide/tab and any corresponding hub component.

In some embodiments, the ID injection shield 760 may comprise one or more axial slots or slits 770. The axial slits 770 may, for example, extend from the distal end of the cylindrical body 762 toward the proximal end of the cylindrical body 762. In some embodiments, the axial slits 770 may provide viewing windows through which lower leg members of a hub nested within the interior volume 762-1 may be visible, *e.g.,* in the case that they align radially with the axial slits 770 (e.g., in the case that a second or third state or engagement position is attained - e.g., during and/or after use/injection).

In some embodiments, fewer or more components 762, 762-1, 762-2, 764, 764-1a, 764-1b, 766, 768, 768-1, 768-2, 770 and/or various configurations of the depicted components 762, 762-1, 762-2, 764, 764-1a, 764-1b, 766, 768, 768-1, 768-2, 770 may be included in the ID injection shield 760 without deviating from the scope of embodiments described herein. In some embodiments, the components 762, 762-1, 762-2, 764, 764-1a, 764-1b, 766, 768, 768-1, 768-2, 770 may be similar in configuration and/or functionality to similarly named and/or numbered components as described herein. In some embodiments, the ID injection shield 760 (and/or portions thereof) may comprise a disposable, auto-disabled, single-dose delivery assembly (or portion thereof) operable to be utilized to execute, conduct, and/or facilitate various methods such as one or more BFS ID injection methods that utilize internal elastic forces to automatically enable and/or disable an injection system based on application of axial force thereto.

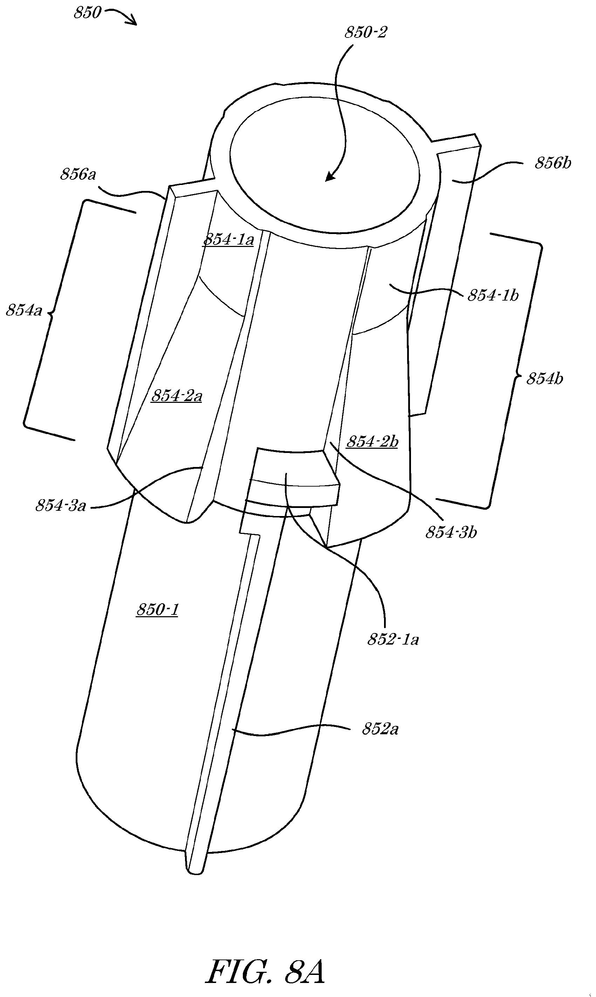

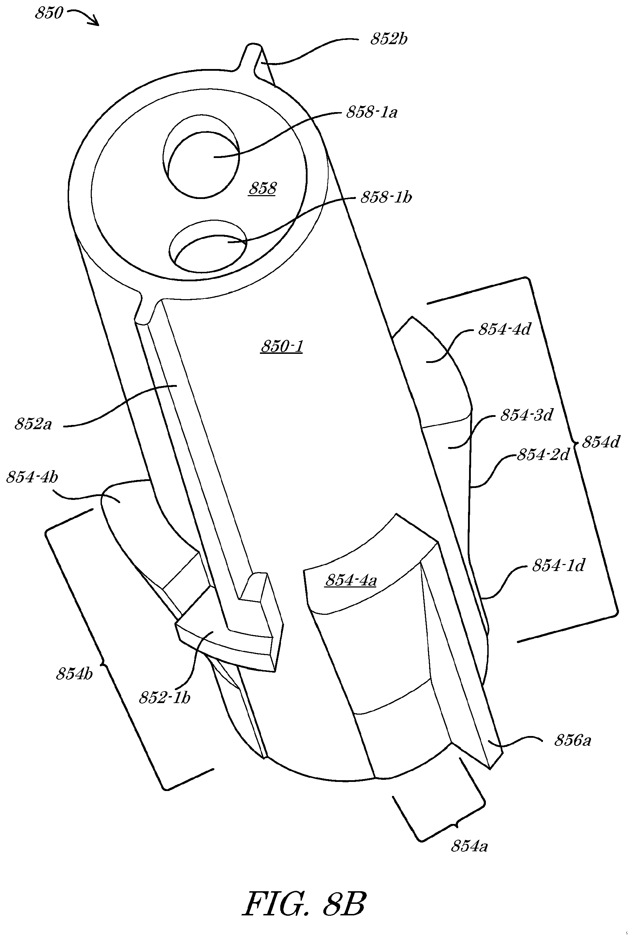

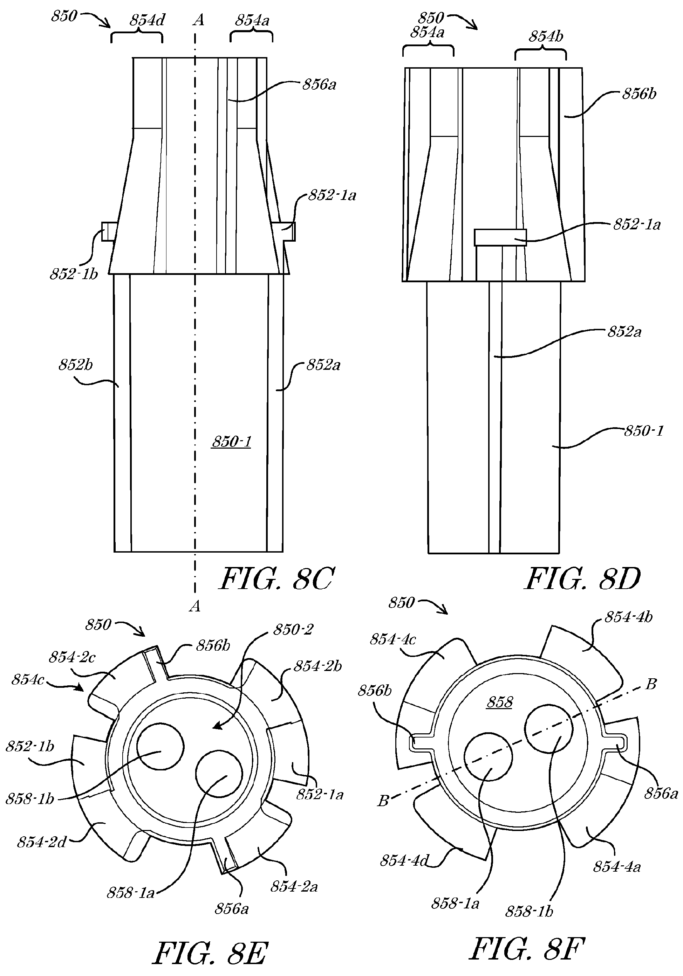

Turning now to FIG. 8A, FIG. 8B, FIG. 8C, FIG. 8D, FIG. 8E, and FIG. 8F, top left perspective, bottom left perspective, front, left, top, and bottom views of a BFS ID injection piston 850 according to some embodiments are shown. In some embodiments, the BFS ID injection piston 850 may comprise similar features and/or configurations and/or may be similar to the piston 650 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein. The BFS ID injection piston 850 may comprise and/or define, for example, a circular, annular, and/or cylindrical body 850-1 defining a piston bore 850-2 therethrough. In some embodiments, the BFS ID injection piston 850 and/or the cylindrical body 850-1 thereof may comprise and/or define one or more axial flanges 852a-b extending from a proximal end of the cylindrical body 850-1 and axially along an outer surface of the cylindrical body 850-1, and protruding radially outward along the length thereof. In some embodiments, the axial flanges 852a-b may terminate at and/or comprise respective flange stops 852-1a, 852-1b. According to some embodiments, the BFS ID injection piston 850 and/or the cylindrical body 850-1 thereof may comprise and/or define one or more leg tracks 854a-d extending from a distal end of the cylindrical body 850-1 and axially along the outer surface of the cylindrical body 850-1.

According to some embodiments, the leg tracks 854a-d may comprise a number of symmetrical and/or symmetrically radially spaced areas of the cylindrical body 850-1 that are configured to accept and/or guide hub legs (not shown; *e.g.,* the lower legs 648 of the hub 640 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). According to some embodiments, each leg track 854a-d may comprise first or upper portion 854-1a, 854-1b, 854-1d, a lower portion 854-2a, 854-2b, 854-2c, 854-2d, an edge portion 854-3a, 854-3b, 854-3d, and/or a bottom portion 854-4a, 854-4b, 854-4d. In some embodiments, the upper portions 854-1a, 854-1b, 854-1d may comprise axial surfaces along the cylindrical body 850-1 that accept and/or guide corresponding hub legs edge portion 854-3a, 854-3b, 854-3d axially along the cylindrical body 850-1. In some embodiments, the upper portions 854-1a, 854-1b, 854-1d may increase the diameter of the cylindrical body 850-1 but may be substantially parallel and/or planar to the outer surface of the cylindrical body 850-1. According to some embodiments, the lower portions 854-2a, 854-2b, 854-2c, 854-2d may comprise contiguous areas along the cylindrical body 850-1 that defines a ramp that constantly increases the outer diameter of the cylindrical body 850-1 along the length of the lower portions 854-2a, 854-2b, 854-2c, 854-2d. In such a manner, continued axial engagement of the hub legs along the leg tracks 854a-d may increasingly deflect and/or bend the hub legs radially outward. In some embodiments, each of the leg tracks 854a-d may be radially disposed about the circumference of the cylindrical body 850-1 to engage with and/or guide the respective hub legs in the case that the hub legs are disposed in a first state or engagement position (*e.g.,* first rotational positions).

According to some embodiments, in the case that the hub legs are rotated to a second or third state or engagement position (*e.g.,* second and/or third rotational positions), the hub legs may rotate off of the leg tracks 854a-d and may become disposed between the leg tracks 854a-d. In some embodiments, the edge portions 854-3a, 854-3b, 854-3d may be shaped to facilitate the falling off/de-railing of the hub legs, *e.g.,* by comprising rounded or chamfered edges or surfaces. According to some embodiments, the edge portions 854-3a, 854-3b, 854-3d may define radial stop surfaces that prevent further rotational movement of the hub legs (*e.g*., locking the hub legs between the leg tracks 854a-d and accordingly preventing subsequent radial deflection/bending of the hub legs by the BFS ID injection piston 850. In some embodiments, the BFS ID injection piston 850 and/or the cylindrical body 850-1 may comprise one or more 856a-b that comprise radial protrusions that similarly prevent or limit radial movement of the hub legs around the circumference of the cylindrical body 850-1. In some embodiments, the flange stops 852-1a, 852-1b may limit axial advancement of the hub legs in one or more of the areas between the leg tracks 854a-d.

According to some embodiments, the proximal end of the cylindrical body 850-1 and the axial flanges 852a-b may be inserted into a shield component and/or a piston bore thereof (not shown; *e.g.,* the piston bore 664 of the injection shield 660 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). In such cases, the limit of axial insertion of the cylindrical body 850-1 into and/or through the piston bore of the shield component may be limited by the bottom portions 854-4a, 854-4b, 854-4d. The bottom portions 854-4a, 854-4b, 854-4d may engage with stop surfaces of the injection shield, for example, preventing insertion of the BFS ID injection piston 850 beyond the extent of the axial flanges 852a-b.

In some embodiments, the proximal or administration end of the BFS ID injection piston 850 may comprise an end surface 858 defining and/or comprising one or more administration or guide holes 858-1a, 858-1b. According to some embodiments, the end surface 858 may comprise a concave surface, *e.g.,* for ID injections, such that an ID bleb may be formed in the concave cavity thereof when the end surface 858 is pressed against the skin for ID injection, thereby allowing the bleb to form without pressure being exerted thereupon by the end surface 858. In some embodiments, the guide holes 858-1a, 858-1b may be disposed eccentric to a central axis "A" of the BFS ID injection piston 850 such that an administration member/needle disposed within the piston bore 850-2 may be selectively aligned or misaligned with the guide holes 858-1a, 858-1b by radial displacement of the axis of the administration member/needle. In some embodiments, two (2) guide holes 858-1a, 858-1b spaced equidistant from the central axis "A" along a common line or axis "B" and may be utilized such that during assembly of a BFS ID injection system including the BFS ID injection piston 850, an administration member/needle may be advantageously positioned in one of two one hundred and eight degree (180°) offset positions/orientations while still permitting proper alignment (or misalignment) with one of the guide holes 858-1a, 858-1b. In such a manner, for example, assembly of system parts may be simplified, reducing system costs.

According to some embodiments, fewer or more components 850, 850-1, 850-2, 852a-b, 852-1a, 852-1b, 854a-d, 854-1a, 854-1b, 854-1d, 854-2a, 854-2b, 854-2c, 854-2d, 854-3a, 854-3b, 854-3d, 854-4a, 854-4b, 854-4d, 856a-b, 858, 858-1a, 858-1b and/or various configurations of the depicted components 850, 850-1, 850-2, 852a-b, 852-1a, 852-1b, 854a-d, 854-1a, 854-1b, 854-1d, 854-2a, 854-2b, 854-2c, 854-2d, 854-3a, 854-3b, 854-3d, 854-4a, 854-4b, 854-4d, 856a-b, 858, 858-1a, 858-1b may be included in the BFS ID injection piston 850 without deviating from the scope of embodiments described herein. In some embodiments, the components 850, 850-1, 850-2, 852a-b, 852-1a, 852-1b, 854a-d, 854-1a, 854-1b, 854-1d, 854-2a, 854-2b, 854-2c, 854-2d, 854-3a, 854-3b, 854-3d, 854-4a, 854-4b, 854-4d, 856a-b, 858, 858-1a, 858-1b may be similar in configuration and/or functionality to similarly named and/or numbered components as described herein. In some embodiments, the BFS ID injection piston 850 (and/or portions thereof) may comprise a disposable, auto-disabled, single-dose delivery assembly (or portion thereof) operable to be utilized to execute, conduct, and/or facilitate various methods such as one or more BFS ID injection methods that utilize internal elastic forces to automatically enable and/or disable an injection system based on application of axial force thereto.

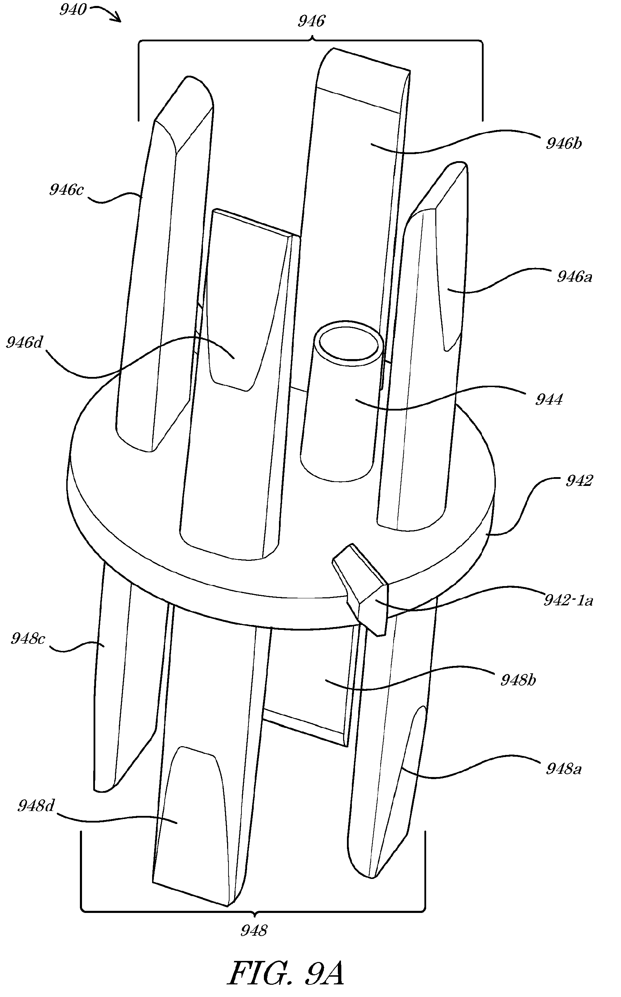

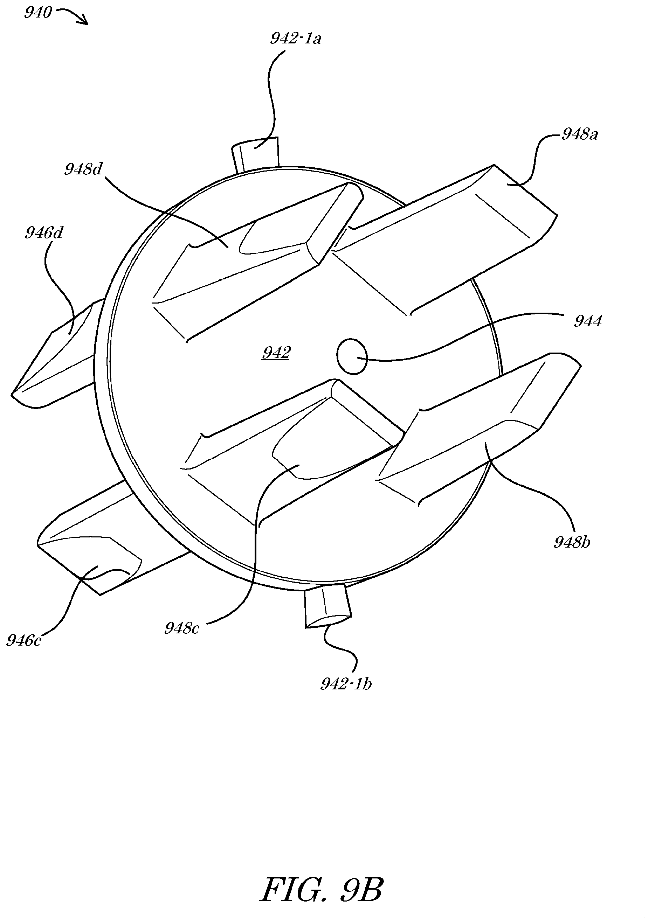

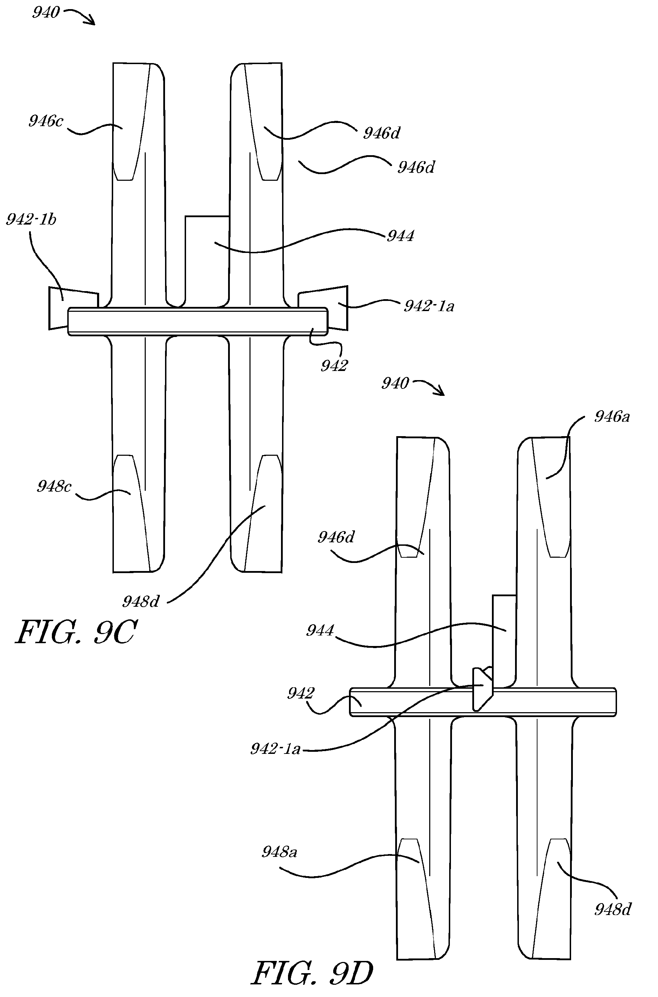

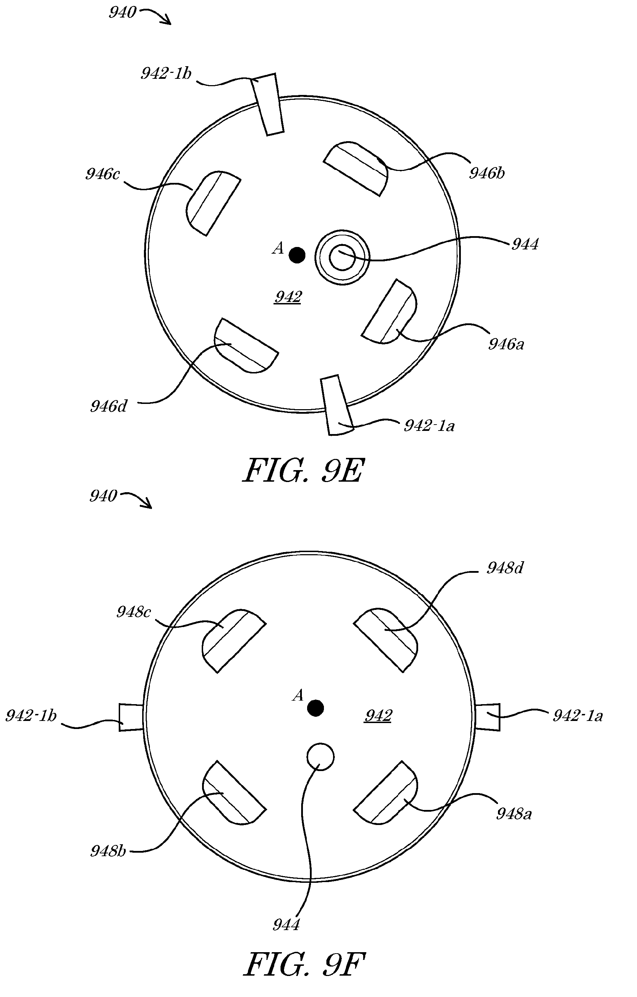

Turning to FIG. 9A, FIG. 9B, FIG. 9C, FIG. 9D, FIG. 9E, and FIG. 9F, top left perspective, bottom left perspective, front, left, top, and bottom views of an ID injection hub 940 according to some embodiments are shown. In some embodiments, the ID injection hub 940 may comprise similar features and/or configurations and/or may be similar to the hub 640 of 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein. The ID injection hub 940 may comprise and/or define, for example, a circular, annular, and/or cylindrical base 942. In some embodiments, the base 942 may comprise one or more radially protruding tabs or base guides 942-1a, 942-1b extending outwardly from an outer radius of the base 942. According to some embodiments, the base guides 942-1a, 942-1b may be shaped with one flat side and one beveled side as depicted, e.g., to effectuate selective rotation of the ID injection hub 940 upon entry and/or exit of the base guides 942-1a, 942-1b into slots or tracks of a connector component (not shown; *e.g.,* the connector component 630 of 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). In some embodiments, an administration seat 944 may extend through the base 942 from a first side to a second side thereof. As depicted, in some embodiments the administration seat 944 may be eccentric to a central axis "A" of the ID injection hub 940, *e.g.,* to facilitate and/or enable auto-disable functionality as described herein. According to some embodiments, the ID injection hub 940 may comprise one or more first or upper legs 946 extending axially away from the base 942 (*e.g.,* in a first axial direction) and/or may comprise one or more second or lower legs 948 extending axially away from the base 942 (*e.g.,* in a second axial direction). According to some embodiments, the legs 946, 948 (of which four (4) of each type are shown for example purposes, namely, four (4) upper legs 946a-d and four (4) lower legs 948a-d) may comprise pliable and/or elastic or semi-elastic elongated members that extend from the base 942 from respective locations radially disposed between the center of the base 942 and the radial extents of the base 942. In some embodiments, each leg 946, 948 may comprise and/or define an outside bevel extending inward axially from the extents/terminus thereof, *e.g.,* to reduce the rigidity of the legs 946, 948 and promote a desired level of elastic spring force capability.

In some embodiments, fewer or more components 942, 942-1a, 942-1b, 944, 946, 946a-d, 948, 948a-d and/or various configurations of the depicted components 942, 942-1a, 942-1b, 944, 946, 946a-d, 948, 948a-d may be included in the ID injection hub 940 without deviating from the scope of embodiments described herein. In some embodiments, the components 942, 942-1a, 942-1b, 944, 946, 946a-d, 948, 948a-d may be similar in configuration and/or functionality to similarly named and/or numbered components as described herein. In some embodiments, the ID injection hub 940 (and/or portions thereof) may comprise a disposable, auto-disabled, single-dose delivery assembly (or portion thereof) operable to be utilized to execute, conduct, and/or facilitate various methods such as one or more BFS ID injection methods that utilize internal elastic forces to automatically enable and/or disable an injection system based on application of axial force thereto.

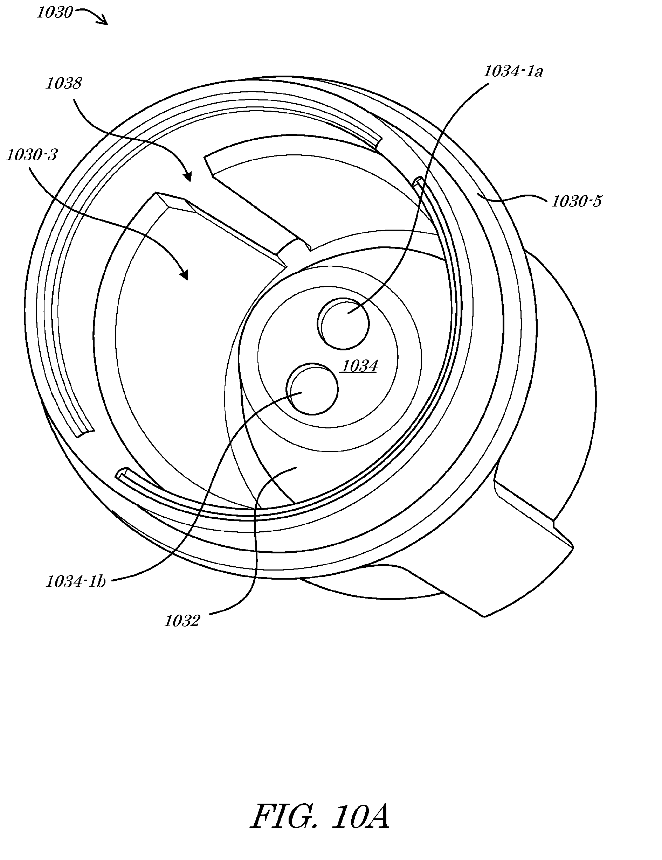

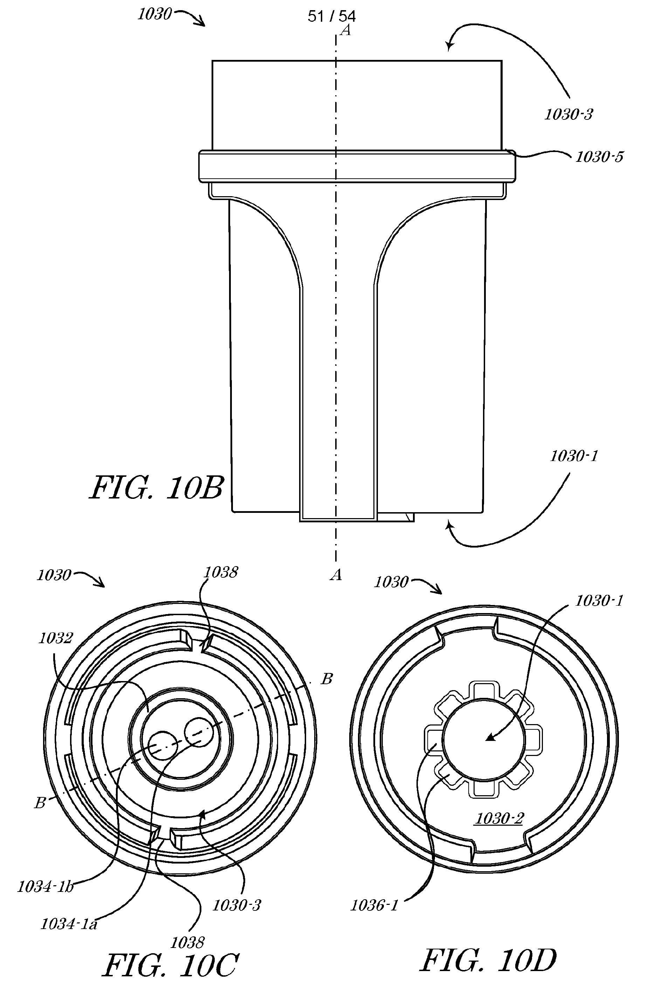

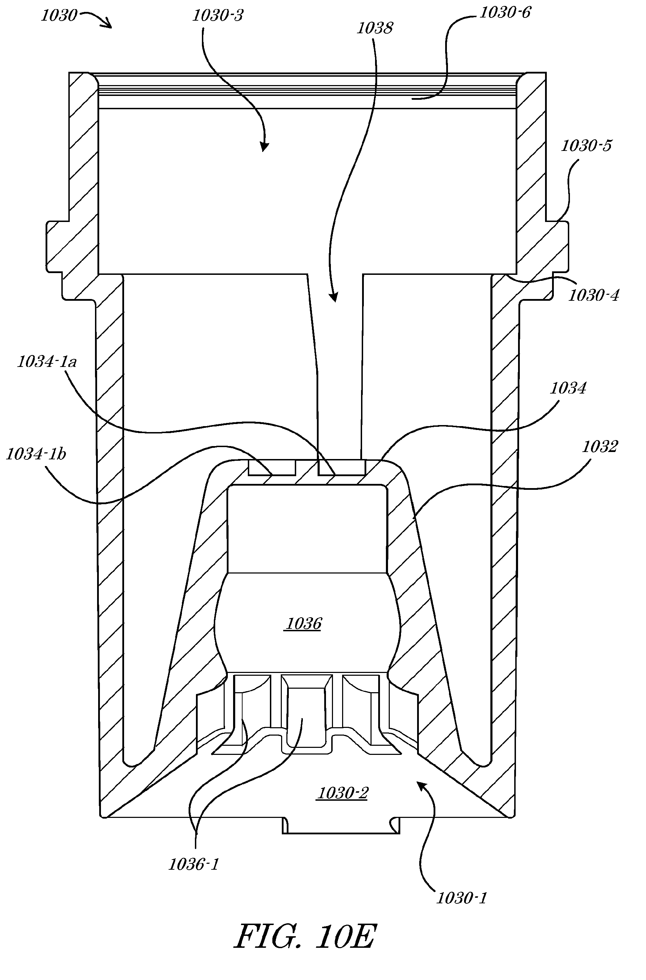

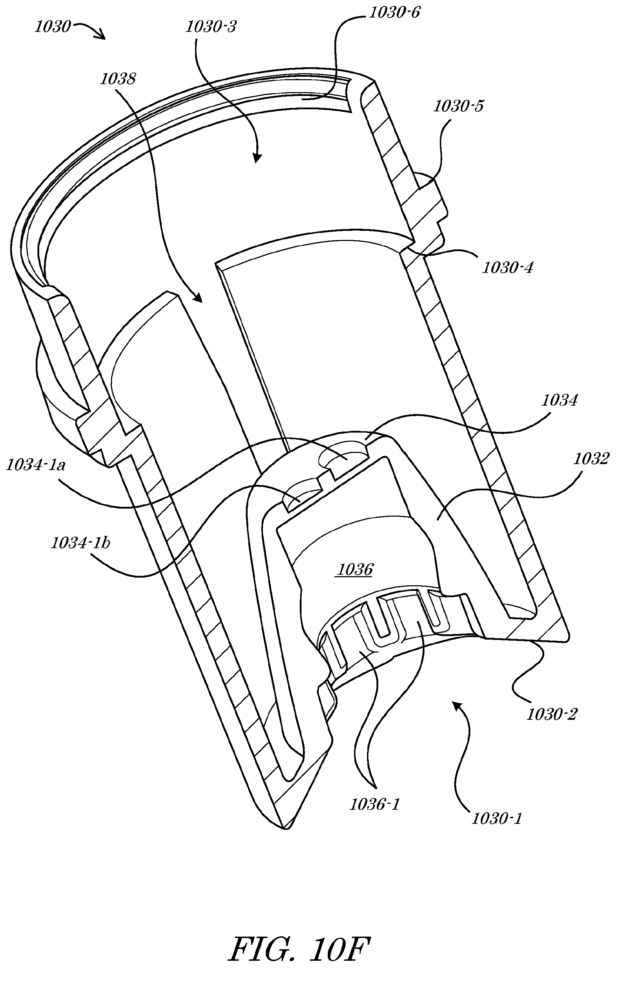

Referring additionally to FIG. 10A, FIG. 10B, FIG. 10C, FIG. 10D, FIG. 10E, and FIG. 10F, bottom left perspective, side, top, bottom, side cross-section, and perspective side cross-section views of a BFS ID injection connector 1030 according to some embodiments are shown. In some embodiments, the BFS ID injection connector 1030 may comprise similar features and/or configurations and/or may be similar to the connector 630 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein. The BFS ID injection connector 1030 may comprise, for example, a generally cylindrical body defining a BFS chamber 1030-1, a BFS detent 1030-2, a hub bore 1030-3, an interior flange 1030-4, and exterior flange 1030-5, and/or an interior groove 1030-6. In some embodiments, a pathway into the BFS chamber 1030-1 may be modified from a simply circular (or other chosen geometry) cross-section to provide for easier entry of an inserted mounting flange of a BFS vial (not shown; *e.g.,* the BFS vial 610 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). The hub bore 1030-3 may also or alternatively be shaped and/or sized to receive and/or engage with various components such as a hub and/or a piston (neither shown; *e.g.,* the hub 640 and/or the piston 650 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein).

In some embodiments, the interior flange 1030-4 may act as an insertion stop that limits the insertion of an injection shield component such as the ID injection shield 660 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein. According to some embodiments, the exterior flange 1030-5 may comprise a lip and/or surface that stops and/or accepts a corresponding surface of a cap (nto shown; *e.g.,* the cap 690 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein) into which the proximal portion of the BFS ID injection connector 1030 is inserted. In some embodiments, the interior groove 1030-6 may be disposed inside the hub bore 1030-3 at the proximal end of the BFS ID injection connector 1030 and be sized and/or shaped to accept a corresponding lip, rib, or rounded flange of an injection shield (not shown; *e.g.,* the ID injection shield 660 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein) coupled to the proximal end of the BFS ID injection connector 1030.

According to some embodiment, the hub bore 1030-3 may comprise and/or define an interior shaped structure such as a pyramid or cone 1032 extending from an interior distal surface of the hub bore 1030-3 and into the hub bore 1030-3. In some embodiments, an axial terminus of the interior cone 1032 may comprise and/or define an interior surface 1034. According to some embodiments, the interior surface 10334 may comprise a planar surface oriented normally to a longitudinal axis "A" of the BFS ID injection connector 1030. In some embodiments, one or more portions of the interior surface 1034 may comprise and/or define a puncture surface 1034-1a, 1034-1b. As depicted in FIG. 10A, for example, the puncture surface(s) 1034-1a, 1034-1b may comprise a circular or cylindrical portion of the interior surface 1034 that comprises an axial thickness that is less than an axial thickness of the remainder of the interior surface 1034. While the interior surface 1034 may comprise and/or define an axial thickness of approximately one millimeter (1-mm) between the hub bore 1030-3 and the BFS chamber 1030-1, for example, the thickness at the puncture surface(s) 1034-1a, 1034-1b may be lesser, such as approximately four tenths of a millimeter (0.4-mm) in thickness. In such a manner, for example, a needle or other puncturing member (not shown) may only successfully puncture the interior surface 1034 through the puncture surface(s) 1034-1a, 1034-1b, while engagement with other portions of the interior surface 1034 would frustrate puncturing, such as by damaging the needle and/or preventing communication from being established between the hub bore 1030-3 and the BFS chamber 1030-1. According to some embodiments, and as depicted, the puncture surface(s) 1034-1a, 1034-1b may be eccentrically situated such that it is offset from the central portion or axis "A" of the BFS ID injection connector 1030. In such a manner, for example, a needle and/or other puncture member that is itself eccentric to the central axis "A" must be rotationally aligned with one of the puncture surface(s) 1034-1a, 1034-1b to permit puncturing/breaching of the interior surface 10334 (and attendant puncturing and/or engagement with a BFS vial (not shown) seated in the BFS chamber 1030-1). In some embodiments, two (2) puncture surfaces 1034-1a, 1034-1b spaced equidistant from the central axis "A" along a common line or axis "B" and may be utilized such that during assembly of a BFS ID injection system including the BFS ID injection connector 1030, an administration member/needle may be advantageously positioned in one of two one hundred and eight degree (180°) offset positions/orientations while still permitting proper alignment (or misalignment) with one of the puncture surfaces 1034-1a, 1034-1b. In such a manner, for example, assembly of system parts may be simplified, reducing system costs.

According to some embodiments, the BFS ID injection connector 1030 and/or the BFS chamber 1030-1 thereof may comprise an internal groove or seat 1036 and/or one or more flange seats 1036-1 that is/are cooperatively sized and configured to receive a mounting flange of a BFS vial (neither shown; *e.g.,* the mounting flange/feature 616 of the BFS vial 610 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). In such a manner, for example, a BFS vial may be selectively and/or removably coupled to the BFS ID injection connector 1030 and, *e.g.,* positioned for selective piercing by an administration member that becomes aligned with and breaches the puncture surface(s) 1034-1a, 1034-1b. According to some embodiments, a cooperatively shaped BFS vial may comprise a neck and exterior flange that fit within the BFS chamber 1030-1 and/or may comprise a conical and/or frustoconical reservoir portion that seats in the BFS detent 1030-2.

In some embodiments, the BFS ID injection connector 1030 may comprise and/or define one or more slots or tracks 1038, *e.g.,* within the hub bore 1030-3. The track(s) 1038 may comprise and/or define, for example, one or more axial grooves, channels, and/or shaped elements that are configured to accept and/or guide one or more corresponding features inserted into the hub bore 1030-3 such as corresponding base guides of a hub component (not shown; *e.g.,* the base guides 642-1a, 642-1b of the hub 640 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). According to some embodiments, the track(s) 1038 may interrupt and/or traverse the interior flange 1030-4, thereby defining an axial pathway through the track(s) 1038 and, *e.g.,* into an engagement with an injection shield inserted into the hub bore 1030-3 (not shown; *e.g.,* the injection shield 660 of FIG. 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). In some embodiments, either or both of the tracks 1038 and the interior cone 1032 may comprise and/or define spiral, helix, diagonal, and/or other non-lineal axial paths, shapes, and/or features that are operable to cause rotation of elements axially engaged therewith.

In some embodiments, fewer or more components 1030-1, 1030-2, 1030-3, 1030-4, 1030-5, 1030-6, 1032, 1034, 1034-1a, 1034-1b, 1036, 1036-1, 1038 and/or various configurations of the depicted components 1030-1, 1030-2, 1030-3, 1030-4, 1030-5, 1030-6, 1032, 1034, 1034-1a, 1034-1b, 1036, 1036-1, 1038 may be included in the BFS ID injection connector 1030 without deviating from the scope of embodiments described herein. In some embodiments, the components 1030-1, 1030-2, 1030-3, 1030-4, 1030-5, 1030-6, 1032, 1034, 1034-1a, 1034-1b, 1036, 1036-1, 1038 may be similar in configuration and/or functionality to similarly named and/or numbered components as described herein. In some embodiments, the BFS ID injection connector 1030 (and/or portions thereof) may comprise a disposable, auto-disabled, single-dose delivery assembly (or portion thereof) operable to be utilized to execute, conduct, and/or facilitate various methods such as one or more BFS ID injection methods that utilize internal elastic forces to automatically enable and/or disable an injection system based on application of axial force thereto.

Turning to FIG. 11A and FIG. 11B, top and bottom perspective views of a BFS ID injection cap 1190 according to some embodiments are shown. In some embodiments, the BFS ID injection cap 1190 may comprise similar features and/or configurations and/or may be similar to the cap 690 of 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein. The BFS ID injection cap 1190 may comprise and/or define, for example, a circular, annular, and/or cylindrical cap body 1190-1 that defines an interior cap volume 1090-2 that is open at a first or distal end thereof. In some embodiments, the interior cap volume 1090-2 may be sized and/or shaped to cover, house, and/or mate with a BFS connector component (not shown; *e.g.,* the connector component 630 of 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein). In some embodiments, the BFS connector component (or a portion threof) may, for example, slide into the interior cap volume 1090-2 and an end wall or edge of the BFS ID injection cap 1190 may be stopped by, seated and/or mated with a corresponding flange surface (not shown; *e.g.,* the exterior flange 630-5 of 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein) of the BFS connector component (*e.g.*, to selectively shield, cover, and/or house the portion of the BFS connector component, an administration member, injection shield, and/or piston component (none of which are shown; *e.g.,* the connector 630, the administration member 680, the injection shield 660, and/or the piston 650 of 6A, FIG. 6B, FIG. 6C, FIG. 6D, FIG. 6E, FIG. 6F, FIG. 6G, FIG. 6H, FIG. 6I, FIG. 6J, FIG. 6K, FIG. 6L, FIG. 6M, and FIG. 6N herein).

In some embodiments, fewer or more components 1190-1, 1190-2 and/or various configurations of the depicted components 1190-1, 1190-2 may be included in the BFS ID injection cap 1190 without deviating from the scope of embodiments described herein. In some embodiments, the components 1190-1, 1190-2 may be similar in configuration and/or functionality to similarly named and/or numbered components as described herein. In some embodiments, the BFS ID injection cap 1190 (and/or portions thereof) may comprise a disposable, auto-disabled, single-dose delivery assembly (or portion thereof) operable to be utilized to execute, conduct, and/or facilitate various methods such as one or more BFS ID injection methods that utilize internal elastic forces to automatically enable and/or disable an injection system based on application of axial force thereto.

### IV. Pre-filled BFS ID Medical Agent Delivery Methods

Various systems and/or components described herein, either alone or in various combinations, may be utilized to execute, conduct, and/or facilitate various methods such as one or more BFS ID injection methods that utilize internal elastic forces to automatically enable and/or disable an injection system based on application of axial force thereto. As described herein, for example, a BFS injection system may comprise internal elastic or semi-elastic legs that are selectively deformed, bent, and/or urged axially outward (*e.g.,* flared) in response to axial compressive force applied to the system components. This may establish internal forces that are utilized to automatically (i) align an administration member with one or more puncture surfaces and/or components such as holes, ports, guides, and/or seals, (ii) puncture one or more seals (*e.g.,* of a BFS vial), and/or (iii) cause a misalignment of one or more components such as the administration member and/or an internal hub component, such misalignment preventing reuse of the system for medical injection (*e.g.,* auto-disable).

In practice, for example, a user may remove a BFS injection system/device from a package and/or seal, remove a cap thereof to expose an injection shield and/or proximal end of an injection piston, and place the proximal end of the injection piston against a target site, such as a human skin area. The user may place a BFS vial in a distal reeving end of the device or the BFS vial may come pre-installed. The user may exert axial force or pressure upon the device by urging the device against the target/skin. In response to the axial force/pressure, the injection piston may move inward into the device, *e.g.,* along with an administration member and/or hub element therein, *e.g.,* keeping the administration member shrouded and/or disengaged from the target. Internal axial movement of the hub element may cause the hub element to undergo a first degree of rotational shift, which may for example, align the administration member with an internal puncture surface, seal, and/or with a seal of the BFS vial. Elastic legs within the device may be forced against one or more radial deflections surfaces such as an interior shaped element and thereby establish an elastic and/or spring force within the device.

Sufficient pressure may be applied by the user to cause the administration member to pierce the internal seal and/or the seal of the BFS vial, thereby placing the contents (*e.g.,* a medication) in fluid communication with the administration member. Continued applied force/pressure may cause the piston element to retract further, exposing an administration end of the administration member. In the case of an ID injection, the administration member/needle may be exposed to approximately one and one half millimeters (1.5-mm). The user may then press a collapsible reservoir of the BFS vial, *e.g.,* against the device (*e.g.,* a detent thereof), thereby forcing the fluid stored therein (or at least a desired dosage portion thereof) through the administration member and into the target/skin. In the case of an IDS injection, the proximal end of the piston may comprise a concave surface that permits an ID bleb to form withint he concave volume while the piston element remains engaged with the target/skin.

The user may withdrawal the device from the target/skin, causing a release of the internal elastic/spring force. The force may be released, for example, by urging guide portions of the hub element to rotate, *e.g.,* by engaging axially with one or more sloped and/or shaped tracks and/or guides that cause the hub to rotate with respect to the piston element. This rotation (which may occur automatically in response to the user removing the axial pressure of the device against the target/skin) may cause the administration member and/or the internal elastic legs to become misaligned, thereby preventing reuse of the device. The device may no longer compress to reveal the administration member due to the elastic legs becoming disengaged and/or locked, for example, and/or the administration member may become misaligned with one or more holes or passages such that it cannot exit the device. In such a manner, for example, the device may automatically manage the internal forces developed by radial deflection of the elastic legs to apply at least one rotational offset that (i) enables the device and/or (ii) disables the device.

Some embodiments may comprise and/or define various systems, methods, articles of manufacture, apparatus, and/or devices that are either stand-alone or may be utilized together. If described as stand-alone, this does not necessarily preclude interoperability with the other disclosed embodiments. Indeed, by being included in the same disclosure, Applicant has anticipated some degree of relation between the disclosed embodiments. If described as cooperative, this does not necessarily preclude stand-alone or alternative operability. Particularly with respect to described systems, for example, while various components are described in relation to their interoperability in some embodiments, in other embodiments one or more of such components may be operative to function without the other (and/or with another component, whether disclosed or not).

Different objects disclosed in different numbered figure sets, for example, may in some cases comprise different inventive components that alone constitute the broadest extents of the disclosure herein (*e.g.,* with or without the other different numbered figure set components). In some embodiments, the combination and/or interaction of a subset of the components may comprise inventive subject matter. The interaction of the hub legs with the cone and/or piston leg tracks may, for example, be inventive with or without any of the other components. Similarly, the automatic misalignment of the needle and/or the legs may be effectuated by a subset of the components without the others being necessary.

While a BFS container having certain features is described and depicted as being part of an injection system, non-BFS vials or different shaped/configured BFS vials may be utilized and/or the BFS vial itself may be inventive without the other components of the system. The BFS vial may be utilized with a different injection connector and/or system, for example. Similarly, the system itself may be utilized without the BFS vial in some embodiments (*e.g.,* the medicament may be otherwise incorporated into the system). In some embodiments, the system may be novel and functional without certain components such as the cap. Similarly, and as noted herein, while some components are described as comprising single or multiple objects, in some embodiments components may be combined to provide the same or similar features in fewer objects or may be split or segmented into more objects. The hub and the piston may be combined to retain the needle, for example, and/or the connector may be incorporated with the shield, without deviating from some embodiments.

### V. Rules of Interpretation

Throughout the description herein and unless otherwise specified, the following terms may include and/or encompass the example meanings provided. These terms and illustrative example meanings are provided to clarify the language selected to describe embodiments both in the specification and in the appended claims, and accordingly, are not intended to be generally limiting. While not generally limiting and while not limiting for all described embodiments, in some embodiments, the terms are specifically limited to the example definitions and/or examples provided. Other terms are defined throughout the present description.

Numerous embodiments are described in this patent application, and are presented for illustrative purposes only. The described embodiments are not, and are not intended to be, limiting in any sense. The presently disclosed invention(s) are widely applicable to numerous embodiments, as is readily apparent from the disclosure. One of ordinary skill in the art will recognize that the disclosed invention(s) may be practiced with various modifications and alterations, such as structural, logical, software, and electrical modifications. Although particular features of the disclosed invention(s) may be described with reference to one or more particular embodiments and/or drawings, it should be understood that such features are not limited to usage in the one or more particular embodiments or drawings with reference to which they are described, unless expressly specified otherwise.

Devices that are in communication with each other need not be in continuous communication with each other, unless expressly specified otherwise.

While threads and/or other specific coupling mechanisms between different components are described for purposes of example herein, fewer, more, and/or different types and/or configurations of coupling mechanisms may be utilized without deviating from some embodiments. While different types and/or configurations of coupling mechanisms may be utilized, in some embodiments those specifically described types and/or configurations of coupling mechanisms may provide advantages such as facilitating the execution of various methods. Additionally, while some components that are coupled together are depicted and/or described as being separate and/or distinct components, in some embodiments two or more coupled and/or mated components may be manufactured and/or provided as a single joint and/or integral component, as is or becomes desirable and/or practicable.

A description of an embodiment with several components or features does not imply that all or even any of such components and/or features are required. On the contrary, a variety of optional components are described to illustrate the wide variety of possible embodiments of the present invention(s). Unless otherwise specified explicitly, no component and/or feature is essential or required.

Further, although process steps, algorithms or the like may be described in a sequential order, such processes may be configured to work in different orders. In other words, any sequence or order of steps that may be explicitly described does not necessarily indicate a requirement that the steps be performed in that order. The steps of processes described herein may be performed in any order practical. Further, some steps may be performed simultaneously despite being described or implied as occurring non-simultaneously (e.g., because one step is described after the other step). Moreover, the illustration of a process by its depiction in a drawing does not imply that the illustrated process is exclusive of other variations and modifications thereto, does not imply that the illustrated process or any of its steps are necessary to the invention, and does not imply that the illustrated process is preferred.

The present disclosure provides, to one of ordinary skill in the art, an enabling description of several embodiments and/or inventions. Some of these embodiments and/or inventions may not be claimed in the present application, but may nevertheless be claimed in one or more continuing applications that claim the benefit of priority of the present application. Applicants intend to file additional applications to pursue patents for subject matter that has been disclosed and enabled but not claimed in the present application.

It will be understood that various modifications can be made to the embodiments of the present disclosure herein without departing from the scope thereof. Therefore, the above description should not be construed as limiting the disclosure, but merely as embodiments thereof. Those skilled in the art will envision other modifications within the scope of the invention as defined by the claims appended hereto.

While several embodiments of the present disclosure have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present disclosure. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used.

Those skilled in the art will recognize, or be able to ascertain using no more than routine experimentation, many equivalents to the specific embodiments of the disclosure described herein. It is, therefore, to be understood that the foregoing embodiments are presented by way of example only and that, within the scope of the appended claims and equivalents thereto, the disclosure may be practiced otherwise than as specifically described and claimed. The present disclosure is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, *i.e.,* elements that are conjunctively present in some cases and disjunctively present in other cases. Other elements may optionally be present other than the elements specifically identified by the "and/or" clause, whether related or unrelated to those elements specifically identified, unless clearly indicated to the contrary.

Reference throughout this specification to "one embodiment" or "an embodiment" means that a particular feature, structure, or characteristic described in connection with the embodiment is included in at least one embodiment. Thus, appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

## Claims

1. A pre-filled blow-fill-seal (BFS) intradermal (ID) injection system (600), comprising:
a BFS bottle (610) defining a collapsible fluid chamber, a neck portion, an exterior flange, and a BFS seal at an end of the neck portion;
a connector (630) defining a first connector end and a second connector end, the first connector end comprising an interior seat into which the exterior flange is axially mated, the second connector end comprising a hub bore in which an interior cone and at least one axial track is disposed;
a hub member (640) disposed in the hub bore, the hub member comprising a hub body from which a first plurality of elongate legs extend axially outward in a first direction and from which a second plurality of elongate legs extend axially outward in a second direction, the hub member comprising an administration member seat disposed axially therethrough and defining at least one circumferential tab cooperatively shaped and engaged with the axial track of the connector, and the first plurality of elongate legs being engaged with the interior cone in a first position;
an administration member (680) retained by the administration member seat and extending through the hub body, the administration member comprising a piercing end and an administration end, with the piercing end being disposed within the hub bore;
a piston (650, 850) defining a piston bore within which the administration end of the administration member is disposed and comprising a plurality of axial leg tracks with which the second plurality of elongate legs are engaged; and
a shield (660) comprising a body and defining an interior volume in which the piston is slidably disposed, the shield defining an administration member bore therethrough and the shield comprising a plurality of internal shoulder elements aligned with the second plurality of elongate legs;
wherein axial force applied to the piston causes the piston and the hub member to advance axially into the hub bore which thereby causes the respective first plurality of elongate legs to travel along an increasing diameter of the interior cone and causes the second plurality of elongate legs to engage with an increasing diameter of the axial leg tracks, which thereby causes the first and second elongate legs to be elastically biased in a radially outward direction, and wherein the advancing of the piston and the hub member causes the piercing end of the administration member to pierce the fluid seal of the BFS bottle.

2. The pre-filled BFS ID injection system (600) of claim 1, wherein the administration member comprises an ID needle (680) and the exterior flange comprises an exterior rounded flange formed on the neck portion.

3. The pre-filled BFS ID injection system (600) of claim 1, wherein the administration member is eccentrically positioned with respect to a longitudinal axis (A) of the system within the hub bore.

4. The pre-filled BFS ID injection system (600) of claim 1, wherein axial advancement of the at least one circumferential tab of the hub member (640) into the axial track of the connector causes the hub member to undergo a first degree of rotation.

5. The pre-filled BFS ID injection system (600) of claim 4, wherein the first degree of rotation places the administration member (680) in alignment with the BFS seal.

6. The pre-filled BFS ID injection system (600) of claim 1, wherein removal of the axial force permits the release of internal forces developed by the biasing of the first and second elongate legs and causes a reverse axial advancement of the at least one circumferential tab of the hub member (640) out of the axial track of the connector.

7. The pre-filled BFS ID injection system (600) of claim 6, wherein the reverse axial advancement of the at least one circumferential tab of the hub member (640) out of the axial track of the connector causes the at least one circumferential tab to engage with the shield (660) and wherein the engagement of the at least one circumferential tab with the shield causes the hub member to undergo a second degree of rotation.

8. The pre-filled BFS ID injection system (600) of claim 7, wherein the second degree of rotation places the administration member (680) in misalignment with the administration member bore of the shield.

9. The pre-filled BFS ID injection system (600) of claim 7, wherein the second degree of rotation places the second plurality of elongate legs in misalignment with the axial leg tracks of the piston (650).

10. The pre-filled BFS ID injection system (600) of claim 1, wherein the axial force applied to the piston further causes, (i) for a first portion of the axial advancement, the first plurality of elongate legs to travel along the increasing diameter of the interior cone and wherein the internal shoulder elements prevent the second plurality of elongate legs from engaging with the increasing diameter of the axial leg tracks, which thereby causes only the first elongate legs to be elastically biased in a radially outward direction, and (ii) for a second portion of the axial advancement, wherein the internal shoulder elements disengage with the second plurality of elongate legs, permitting the second plurality of elongate legs to engage with the increasing diameter of the axial leg tracks, which thereby causes a least the second elongate legs to be elastically biased in a radially outward direction.

## Patentansprüche

1. Vorbefülltes intradermales (ID) Blow-Fill-Versiegelungs(BFS)-Injektionssystem (600), das Folgendes umfasst:
eine BFS-Flasche (610), die eine zusammenschiebbare Fluidkammer, einen Halsabschnitt, einen Außenflansch und eine BFS-Versiegelung an einem Ende des Halsabschnitts definiert;
einen Verbinder (630), der ein erstes Verbinderende und ein zweites Verbinderende definiert, wobei das erste Verbinderende einen Innensitz umfasst, in den der Außenflansch axial eingepasst ist, wobei das zweite Verbinderende eine Nabenbohrung umfasst, in der ein Innenkonus und zumindest eine Axialführung angeordnet sind;
ein Nabenelement (640), das in der Nabenbohrung angeordnet ist, wobei das Nabenelement einen Nabenkörper umfasst, von dem aus sich eine Vielzahl von länglichen Schenkeln axial nach außen in eine erste Richtung erstreckt und von dem aus sich eine zweite Vielzahl von länglichen Schenkeln axial nach außen in eine zweite Richtung erstreckt, wobei das Nabenelement einen Verabreichungselementsitz umfasst, der axial durch dieses hindurch angeordnet ist und zumindest eine Umfangslasche definiert, die mit der Axialführung des Verbinders zusammenwirkend geformt und damit in Eingriff ist, und wobei die erste Vielzahl von länglichen Schenkeln an einer ersten Position mit dem Innenkonus in Eingriff gebracht ist;
ein Verabreichungselement (680), das durch den Verabreichungselementsitz zurückgehalten wird und sich durch den Nabenkörper erstreckt, wobei das Verabreichungselement ein Stechende und ein Verabreichungsende umfasst, wobei das Stechende innerhalb der Nabenbohrung angeordnet ist;
einen Kolben (650, 850), der eine Kolbenbohrung definiert, in der das Verabreichungsende des Verabreichungselements angeordnet ist, und der eine Vielzahl von axialen Schenkelführungen umfasst, mit denen die zweite Vielzahl von länglichen Schenkeln in Eingriff gebracht ist; und
ein Schutzelement (660), das einen Körper umfasst und ein Innenvolumen definiert, in dem der Kolben gleitbar angeordnet ist, wobei das Schutzelement eine Verabreichungselementbohrung durch dieses hindurch definiert und das Schutzelement eine Vielzahl von internen Schulterelementen umfasst, die mit der zweiten Vielzahl von länglichen Schenkeln fluchtend ausgerichtet ist;
wobei eine Axialkraft, die auf den Kolben ausgeübt wird, bewirkt, dass der Kolben und das Nabenelement sich axial in die Nabenbohrung vorwärtsbewegen, wodurch bewirkt wird, dass sich eine entsprechende erste Vielzahl von länglichen Schenkeln entlang eines zunehmenden Durchmessers des Innenkonus bewegt und bewirkt, dass die zweite Vielzahl von länglichen Schenkeln mit einem zunehmenden Durchmesser der axialen Schenkelführungen in Eingriff tritt, wodurch bewirkt wird, dass die ersten und die zweiten länglichen Schenkel elastisch in eine radiale Auswärtsrichtung vorgespannt werden, und wobei das Vorwärtsbewegen des Kolbens und des Nabenelements bewirkt, dass das Stechende des Verabreichungselements die Fluidversiegelung der BFS-Flasche durchsticht.

2. Vorbefülltes ID-BFS-Injektionssystem (600) nach Anspruch 1, wobei das Verabreichungselement eine ID-Nadel (680) umfasst und der Außenflansch einen abgerundeten Außenflansch umfasst, der am Halsabschnitt ausgebildet ist.

3. Vorbefülltes ID-BFS-Injektionssystem (600) nach Anspruch 1, wobei das Verabreichungselement exzentrisch in Bezug auf eine Längsachse (A) des Systems innerhalb der Nabenbohrung angeordnet ist.

4. Vorbefülltes ID-BFS-Injektionssystem (600) nach Anspruch 1, wobei das axiale Vorwärtsbewegen der zumindest einen Umfangslasche des Nabenelements (640) in die Axialführung des Verbinders bewirkt, dass das Nabenelement einen ersten Rotationsgrad durchläuft.

5. Vorbefülltes ID-BFS-Injektionssystem (600) nach Anspruch 4, wobei der erste Rotationsgrad das Verabreichungselement (680) mit der BFS-Versiegelung fluchtend ausrichtet.

6. Vorbefülltes ID-BFS-Injektionssystem (600) nach Anspruch 1, wobei ein Wegfall der Axialkraft die Freisetzung von internen Kräften ermöglicht, die durch das Vorspannen der ersten und zweiten länglichen Schenkel entstanden sind, und ein umgekehrtes axiales Vorwärtsbewegen der zumindest einen Umfangslasche des Nabenelements (640) aus der Axialführung des Verbinders heraus bewirkt.

7. Vorbefülltes ID-BFS-Injektionssystem (600) nach Anspruch 6, wobei das umgekehrte axiale Vorwärtsbewegen der zumindest einen Umfangslasche des Nabenelements (640) aus der Axialführung des Verbinders heraus bewirkt, dass die zumindest eine Umfangslasche mit dem Schutzelement (660) in Eingriff tritt, und wobei das Eingreifen der zumindest einen Umfangslasche mit dem Schutzelement bewirkt, dass das Nabenelement einen zweiten Rotationsgrad durchläuft.

8. Vorbefülltes ID-BFS-Injektionssystem (600) nach Anspruch 7, wobei der zweite Rotationsgrad das Verabreichungselement (680) in Fehlausrichtung mit der Verabreichungselementbohrung des Schutzelements bringt.

9. Vorbefülltes ID-BFS-Injektionssystem (600) nach Anspruch 7, wobei der zweite Rotationsgrad die zweite Vielzahl von länglichen Schenkeln in Fehlausrichtung mit den axialen Schenkelführungen des Kolbens (650) bringt.

10. Vorbefülltes ID-BFS-Injektionssystem (600) nach Anspruch 1, wobei die an den Kolben angelegte axiale Kraft außerdem bewirkt, dass (i) sich die erste Vielzahl von länglichen Schenkeln in einem ersten Abschnitt des axialen Vorwärtsbewegens entlang des zunehmenden Durchmessers des Innenkonus bewegt, wobei die internen Schulterelemente verhindern, dass die zweite Vielzahl von länglichen Schenkeln mit dem zunehmenden Durchmesser der axialen Schenkelführungen in Eingriff tritt, wodurch bewirkt wird, dass nur die ersten länglichen Schenkel in eine radiale Auswärtsrichtung vorgespannt werden, und (ii) in einem zweiten Abschnitt des axialen Vorwärtsbewegens, bei dem die internen Schulterelemente den Eingriff mit der zweiten Vielzahl von länglichen Schenkeln lösen, die zweite Vielzahl von länglichen Schenkeln mit dem zunehmenden Durchmesser der axialen Schenkelführungen in Eingriff treten kann, wodurch bewirkt wird, dass zumindest die zweiten länglichen Schenkel in eine radiale Auswärtsrichtung elastisch vorgespannt werden.

## Revendications

1. Système d'injection intradermique (ID) à formage-remplissage-scellage (BFS) prérempli (600), comprenant :
un flacon BFS (610) définissant une chambre de fluide pouvant être aplatie, une partie de col, une bride extérieure et un joint d'étanchéité BFS à une extrémité de la partie de col ;
un connecteur (630) définissant une première extrémité de connecteur et une seconde extrémité de connecteur, la première extrémité de connecteur comprenant un siège intérieur dans lequel la bride extérieure est appariée axialement, la seconde extrémité de connecteur comprenant un alésage de moyeu dans lequel un cône intérieur et au moins une piste axiale sont disposés ;
un élément de moyeu (640) disposé dans l'alésage de moyeu, l'élément de moyeu comprenant un corps de moyeu à partir duquel une première pluralité de pattes allongées s'étendent axialement vers l'extérieur dans une première direction et à partir duquel une seconde pluralité de pattes allongées s'étendent axialement vers l'extérieur dans une seconde direction, l'élément de moyeu comprenant un siège d'élément d'administration disposé axialement à travers celui-ci et définissant au moins une languette circonférentielle formée de manière coopérative et en prise avec la piste axiale du connecteur, et la première pluralité de pattes allongées étant en prise avec le cône intérieur dans une première position ;
un élément d'administration (680) retenu par le siège d'élément d'administration et s'étendant à travers le corps de moyeu, l'élément d'administration comprenant une extrémité de perçage et une extrémité d'administration, l'extrémité de perçage étant disposée à l'intérieur de l'alésage de moyeu ;
un piston (650, 850) définissant un alésage de piston à l'intérieur duquel l'extrémité d'administration de l'élément d'administration est disposée, et comprenant une pluralité de pistes de pattes axiales avec lesquelles la seconde pluralité de pattes allongées sont en prise ; et
une protection (660) comprenant un corps et définissant un volume intérieur dans lequel le piston est disposé de manière coulissante, la protection définissant un alésage d'élément d'administration à travers celui-ci, et la protection comprenant une pluralité d'éléments d'épaulement internes alignés avec la seconde pluralité de pattes allongées ;
dans lequel une force axiale appliquée au piston amène le piston et l'élément de moyeu à avancer axialement dans l'alésage de moyeu, ce qui amène ainsi la première pluralité respective de pattes allongées à se déplacer le long d'un diamètre croissant du cône intérieur et amène la seconde pluralité de pattes allongées à venir en prise avec un diamètre croissant des pistes de pattes axiales, ce qui amène ainsi les premières et secondes pattes allongées à être sollicitées élastiquement dans une direction radialement vers l'extérieur, et dans lequel l'avancée du piston et de l'élément de moyeu amène l'extrémité de perçage de l'élément d'administration à percer le joint d'étanchéité au fluide du flacon BFS.

2. Système d'injection ID BFS prérempli (600) selon la revendication 1, dans lequel l'élément d'administration comprend une aiguille ID (680) et la bride extérieure comprend une bride arrondie extérieure formée sur la partie de col.

3. Système d'injection ID BFS prérempli (600) selon la revendication 1, dans lequel l'élément d'administration est positionné de manière excentrique par rapport à un axe longitudinal (A) du système à l'intérieur de l'alésage de moyeu.

4. Système d'injection ID BFS prérempli (600) selon la revendication 1, dans lequel l'avancée axiale de la au moins une patte circonférentielle de l'élément de moyeu (640) dans la piste axiale du connecteur amène l'élément de moyeu à subir un premier degré de rotation.

5. Système d'injection ID BFS prérempli (600) selon la revendication 4, dans lequel le premier degré de rotation place l'élément d'administration (680) en alignement avec le joint d'étanchéité BFS.

6. Système d'injection ID BFS prérempli (600) selon la revendication 1, dans lequel une élimination de la force axiale permet la libération de forces internes développées par la sollicitation des premières et secondes pattes allongées et provoque une avancée axiale inverse de la au moins une languette circonférentielle de l'élément de moyeu (640) hors de la piste axiale du connecteur.

7. Système d'injection ID BFS prérempli (600) selon la revendication 6, dans lequel l'avancée axiale inverse de la au moins une languette circonférentielle de l'élément de moyeu (640) hors de la piste axiale du connecteur amène la au moins une languette circonférentielle à venir en prise avec la protection (660), et dans lequel la mise en prise de la au moins une languette circonférentielle avec la protection amène l'élément de moyeu à subir un second degré de rotation.

8. Système d'injection ID BFS prérempli (600) selon la revendication 7, dans lequel le second degré de rotation place l'élément d'administration (680) en désalignement avec l'alésage d'élément d'administration de la protection.

9. Système d'injection ID BFS prérempli (600) selon la revendication 7, dans lequel le second degré de rotation place la seconde pluralité de pattes allongées en désalignement avec les pistes de pattes axiales du piston (650).

10. Système d'injection ID BFS prérempli (600) selon la revendication 1, dans lequel la force axiale appliquée au piston amène en outre, (i) pour une première partie de l'avancée axiale, la première pluralité de pattes allongées à se déplacer le long du diamètre croissant du cône intérieur, et dans lequel les éléments d'épaulement internes empêchent la seconde pluralité de pattes allongées de venir en prise avec le diamètre croissant des pistes de pattes axiales, ce qui amène ainsi uniquement les premières pattes allongées à être sollicitées de manière élastique dans une direction radialement vers l'extérieur, et (ii) pour une seconde partie de l'avancée axiale, dans lequel les éléments d'épaulement internes se désengagent de la seconde pluralité de pattes allongées, en permettant à la seconde pluralité de pattes allongées de venir en prise avec le diamètre croissant des pistes de pattes axiales, ce qui amène ainsi au moins les secondes pattes allongées à être sollicitées de manière élastique dans une direction radialement vers l'extérieur.
